(19)

Europäisches
Patentamt
European
Patent Office
Office européen
des brevets

(11)  **EP 3 009 415 A1**

(12)  **EUROPEAN PATENT APPLICATION**
published in accordance with Art. 153(4) EPC

(43) Date of publication:
**20.04.2016  Bulletin 2016/16**

(21) Application number: **14772264.9**

(22) Date of filing: **02.06.2014**

(51) Int Cl.:
*C04B 33/04* (2006.01)      *C04B 33/13* (2006.01)
*C04B 33/32* (2006.01)

(86) International application number:
**PCT/KR2014/004877**

(87) International publication number:
**WO 2014/200218 (18.12.2014 Gazette 2014/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority:  **11.06.2013  KR 20130066702**

(71) Applicant: **Lee, Jong Doo
Anyang Si, Gyeonggi-do 431-070 (KR)**

(72) Inventors:
• **RHEE, shang hi
  Seoul 135-743 (KR)**
• **BAHNG, gun woong
  Daejeon 302-806 (KR)**

(74) Representative: **von Kreisler Selting Werner -
Partnerschaft
von Patentanwälten und Rechtsanwälten mbB
Deichmannhaus am Dom
Bahnhofsvorplatz 1
50667 Köln (DE)**

(54)  **METHOD FOR EXTRACTING MINERAL SOMATID AND METHOD FOR PREPARING
MULTIFUNCTIONAL ADVANCED MATERIALS USING SAME**

(57)      A method of manufacturing a healing and multifunctional natural gel using mineral somatid quantum energy living bodies (QELBs). The composite material including mineral somatid is produced by separating mineral somatid from the mineral, culturing the separated mineral somatid, and combining the cultured mineral somatid with powder. The composite material is harmless, has no biological side effects, has multiple functions including cancer suppression, immune enhancement, skin reconstruction, self-heating, cold activeness, VOC deodorization and harmful electromagnetic radiation shielding and absorption, and serves to increase flavor and beneficial components by promoting fermentation and ripening.

[Fig 1]

**Description**

**TECHNICAL FIELD**

The present invention relates to a method of extracting mineral somatid and a method of manufacturing an advanced multifunctional material using the same. More particularly, the present invention relates to a method of extracting mineral somatid and a method of manufacturing an advanced multifunctional material using the same in which the mineral somatid is cultured after being separated from the mineral and the resultant material is manufactured using the cultured mineral somatid. The resultant material is harmless, has no biological side effects, and has multiple functions including cancer suppression, immune enhancement, skin reconstruction, self-heating, cold activeness, VOC deodorization and harmful electromagnetic radiation shielding and absorption.

**BACKGROUND ART**

Bechamp, a contemporary medical scientist with Pasteur, discovered microscopic living bodies that cause fermentation and named them microzya, which means "tiny bodies" or "ferment substances." Bechamp explained microzyma as living bodies that cause fermentation, that is, fermentation does not occur without microzyma. In addition, microzyma do not exist in a purely artificial material. Microzyma are involved in the process of creation and destruction in nature without dying, and are an independent anatomic element that exists in a living body as a third component of the blood.

Bechamp also explained that microzyma exist in natural minerals. However, neither characteristics nor applications of microzyma except for fermentation have been known.

After Bechamp, similar studies were reported by Gunther Enderlein, Claude Bernard, Wilhelm Reich, Royal Reif, Virginia Livingston-Wheeler and others. Doctor Gaston Naessens in Canada discovered very tiny living bodies while he was observing the blood using a special optical microscope that he developed, which has a magnification power of 30,000X or greater, and named them somatid.

Gaston Naessens published that somatid has the following characteristics based on the results that he had researched for tens of years through his lifetime.

1. The somatid is a precursor of deoxyribonucleic acid (DNA). This indicates that somatid is a link between a molecule of a substance and DNA that exhibits the characteristics of a living organism.

2. Virus is believed to mediate between a substance and a living body, and its life cannot exist without a host. In contrast, somatid can independently exist without nutrition from the outside. Somatid is characterized to survive both in vivo and in vitro.

3. Somatid is a concretization of energy, which is essential for forming trephine that stimulates cell division. This means cell division is impossible without the existence of somatid. Therefore, somatid is a source of life.

4. Somatid has genetic information although it has neither DNA nor ribonucleic acid (RNA). While viruses have a DNA particle or a DNA fragment, somatid is a precursor that induces the formation of DNA.

5. Somatid basically has electrical characteristics, in which the nucleus of somatid is charged positively, and the film of somatid is charged negatively. Although the characteristics of a somatid are similar to those of a cell, somatid is a basic unit of a living organism with a size of $0.2\mu m$ or less, which is smaller than the cell.

6. Somatid is the tiniest living condenser of energy, and is more basic living unit than the stem cell. Therefore, somatid can be applied like the stem cell.

7. Somatid is subcellular. Somatid can reproduce itself, live at a high temperature of 200°C or higher where carbonization occurs, endure strong acid, and persist under radioactive radiation of 50,000rem. In a dry condition, somatid becomes a crystal shape. In this condition, somatid is too strong to be cut by any kind of knives. It stays in a form similar to a spore for millions of years until a suitable is reached.

A method of collecting somatid that provides useful function to the human body is disclosed in Japanese Laid-Open Patent Publication No. 2006-166738 (June 29, 2006).

This earlier-filed patent publication acquires somatid by a method for collecting ancient somatid. The collecting method is characterized by the process of making fossil seashell into fine particles having a particle diameter ranging from 0.1 to $5\mu m$, the process of mixing 1g of fine particles of the shell fossil with 5cc of water, the process of possessing a mixed solution of fine particles and water for at least 4 hours, and the process of separating supernatant liquid from precipitate having calcium carbonate as a main component to give the supernatant liquid as water containing ancient somatid.

In this related art, however, sources from which somatid is to acquired are limited to ancient shell fossil, and the amount of somatid that is acquired is very small. Although the acquired somatid may be used for the purpose of pure research, it is difficult to use the acquired somatid for a material necessary for actual life.

In some aspects, somatid as described above is similar to cell proliferation by Bonghan ducts and Sanal, which have been studied by the team of professor Kwang Sup Soh of Seoul National University. Recently, the team of professor Kwang Sup Soh published a number of reports about Bonghan ducts and Sanal.

The foregoing studies of the related art have been focused on somatid present in the living bodies of men, plants or animals. Considering the characteristics of biological environment, it is difficult to culture or extract a large amount of somatid, and somatid cannot be used as a composite material.

## DISCLOSURE

## Technical Problem

The present invention has been made keeping in mind the above problems occurring in the prior art, and an object of the present invention is to provide a composite material including mineral somatid by separating mineral somatid from the mineral, culturing the separated mineral somatid, and combining the cultured mineral somatid with a natural plant extract. The composite material according to the present invention is harmless, has no biological side effects, and has multiple functions including cancer suppression, immune enhancement, skin reconstruction, self-heating, cold activeness, VOC deodorization and harmful electromagnetic radiation shielding and absorption.

The present invention is also intended to allow an advanced multifunctional composite material to be mass-produced more easily and at a low cost by mixing a natural plant extract composed of various kinds of microbial materials with quantum energy living bodies (QELBs, or mineral somatid) that show active signs of life, have multiple functions, such as healing, and can persist when heated at a high temperature of 1,000°C for 10 hours.

The present invention is also intended to produce medicines, bio products and foods that are used for cancer, diabetes, blood pressure disorders, apoplexy, acquired immune deficiency syndrome (AIDS), Parkinsonism, metabolic diseases, cardiac disorders and organ transplants using an advanced multifunctional composite material in which mineral somatid having superior dispersibility is contained.

The present invention is also intended to produce coating materials that can block and absorb radio waves and disperse heat, composite materials for military weapons and non-weapons, and composite materials for spacecrafts, aircrafts, containers and vehicles using an advanced multifunctional composite material in which mineral somatid having superior dispersibility is contained.

The present invention is also intended to produce construction materials, such as cement, tiles, bricks, paints, heat retention materials, insulators, flooring, windows and doors, soil conditioners, environmental-friendly fertilizers, agricultural chemicals, and agricultural materials, such as vinyl heat retention materials, using an advanced multifunctional composite material in which mineral somatid having superior dispersibility is contained.

The present invention is also intended to produce materials that can improve water quality, reduce green algae, treat wastewater, purify toxic exhaust gases, reduce whitening and purify oils using an advanced multifunctional composite material in which mineral somatid having superior dispersibility is contained.

The present invention is also intended to produce advanced composite materials for information technologies, electronics, semiconductor, liquid-crystal displays (LCDs), light-emitting diodes (LEDs) and three-dimensional (3D) technologies using an advanced multifunctional composite material in which mineral somatid having superior dispersibility is contained.

The present invention is also intended to produce fibers, clothes, chemicals and polymeric materials using an advanced multifunctional composite material in which mineral somatid having superior dispersibility is contained.

## Technical Solution

In order to accomplish the foregoing objects, the present invention provides a method of extracting mineral somatid. The method includes the following steps of: (a) mining a natural mineral under the earth, wherein the natural mineral does not have heavy meals or a radioactive substance harmful to living bodies and is not contaminated; (b) crushing the mined natural mineral into powder having a particle size ranging from 320 meshes to 2 nanometers; (c) refining the mineral powder by removing heavy metals and radioactive substances that are harmful to living bodies from the mineral powder; (d) burning the refined mineral powder in order to change a mass, a specific gravity, a number of electrons and an ion number of the refined mineral powder; (e) mixing the burned mineral powder with $H_2O$ and a natural plant extract at certain ratios, and fermenting a mineral powder mixture for a predetermined period so that mineral somatid and microorganisms are activated; (f) sterilizing and drying the fermented mineral powder mixture; (g) culturing the mineral powder mixture by adding a solution to the dried mineral powder mixture and adding a nutrient medium to the mineral powder mixture to which the solution is added; and (h) extracting microorganisms from the mineral powder mixture cultured at the step (g), and extracting mineral somatid from the extracted microorganisms.

In the method of extracting mineral somatid according to the present invention, the natural mineral may be a mineral which contains inorganic matters of $SiO_2$, $Al_2O_3$, $Fe_2O_3$ and $MgO_3$.

In the method of extracting mineral somatid according to the present invention, the step (d) may include a first burning process of calcining the mineral powder by heating the mineral powder at a temperature ranging from 50 to 300°C for 2 to 3 hours; and after the first burning process is completed, a second burning process of burning the mineral powder that has been burned in the first burning process by heating the mineral powder at a temperature ranging from 300 to 850°C for 30 minutes to 10 hours.

In the method of extracting mineral somatid according to the present invention, the step (e) may include forming the mineral powder mixture by mixing 70% the mineral powder that has been crushed at a size ranging from 320 meshes to 2 nanometers, 25% $H_2O$ and 5% a nontoxic plant extract of roots, stems or leaves.

In the method of extracting mineral somatid according to the present invention, the step (e) may include putting the mineral powder mixture into a container, and ripening and fermenting the mineral powder mixture in a ripening chamber at a temperature ranging from -10 to 200 °C for 10 to 90 days so that the mineral somatid and microorganisms are activated.

In the method of extracting mineral somatid according to the present invention, the mineral powder of the mineral powder mixture may be crushed until a size of the mineral powder ranges from 320 meshes to 2 nanometers so that only the powder from which heavy metals and harmful components are removed is extracted for use.

In the method of extracting mineral somatid according to the present invention, the step (f) may include rotating and sterilizing the mineral powder mixture inside a calciner at a high temperature of 180°C or below for 30 minutes to 2 hours, and drying the sterilized mineral powder mixture at a temperature ranging from 150 to 200°C.

In the method of extracting mineral somatid according to the present invention, the step (g) may include putting the dried mineral powder mixture into a container, adding a solution that includes a predetermined amount of water, distilled water, magnetized water, glucose or sugar to the mineral powder mixture, adding sugar or a nutrient medium for the culturing of microorganisms to the mineral powder mixture to which the solution is added, and culturing the mineral powder mixture for at least one hour.

In the method of extracting mineral somatid according to the present invention, conditions for culturing the mineral powder mixture may include a culture medium selected from the group consisting of yeast extraction mineral medium (YEM), tryptic soy broth (TSB) medium, M9 medium and Luria broth (LB) medium and a culturing temperature ranges from 30 to 37°C, a composition of yeast extraction mineral medium (YEM) consists of $Na_2HPO_4 \cdot 12H_2O$ 3.5g, $K_2HPO_4$ 1.0g, $MgSO_4 \cdot 7H_2O$ 0.03g, $NH_4Cl$ 0.5g, yeast extract 4.0g, agar 15.0g, and distilled water 1.0L.

In the method of extracting mineral somatid according to the present invention, the step of extracting the mineral somatid may include extracting only mineral somatid that continues life activity and radiates energy after being sterilized at a high temperature of 1,000°C or higher for at least 10 hours in an autoclave.

## Advantageous Effects

According to the present invention as set forth above, the composite material including mineral somatid is produced by separating mineral somatid from the mineral, culturing the separated mineral somatid, and combining the cultured mineral somatid with powder. The composite material is harmless, has no biological side effects, has multiple functions including cancer suppression, immune enhancement, skin reconstruction, self-heating, cold activeness, VOC deodorization and harmful electromagnetic radiation shielding and absorption, and serves to increase flavor and beneficial components by promoting fermentation and ripening.

In addition, according to the present invention, the advanced multifunctional composite material can be mass-produced more easily and at a low cost by mineral somatid or mixing quantum energy living bodies (QELBs) that show active signs of life and have multiple functions, such as healing, with a ceramic powder composed of various kinds of microbial materials which can persist when heated at a high temperature of 1,000°C for 10 hours.

Furthermore, according to the present invention, the multifunctional composite material in which the mineral somatid having superior dispersibility is contained can be easily combined with fiber and a polymeric material, such as chemicals. It is also possible to develop new composite materials by combining the multifunctional composite material with industrial materials such as iron, nonferrous materials and ceramics.

In addition, according to the present invention, the multifunctional composite material acts to neither attack nor kill cells or microorganisms that are beneficial or not harmful to living bodies while protecting them to survive at high temperature or in the hostile environment, and suppresses pathogenic bacteria that are harmful to living bodies or super bacteria resistant to antibiotics (antibiosis).

Furthermore, according to the present invention, the multifunctional composite material can serve as catalyst that enhances the intrinsic properties of a material by being combined with a variety of materials, such as polymeric materials, metal materials, inorganic materials or organic materials, and can suppress oxidation while enabling reduction.

In addition, according to the present invention, it is possible to produce medicines, bio products and foods that are used for cancer, diabetes, blood pressure disorders, apoplexy, acquired immune deficiency syndrome (AIDS), Parkinsonism, metabolic diseases, cardiac disorders and organ transplants using the advanced multifunctional composite material in which mineral somatid having superior dispersibility is contained, thereby healing not only humans but also animals and plants so that they can stay healthy.

Furthermore, according to the present invention, it is possible to produce coating materials that can block and absorb radio waves and disperse heat, composite materials for military weapons and non-weapons, and composite materials for spacecrafts, aircrafts, containers and vehicles using the advanced multifunctional composite material in which mineral somatid having superior dispersibility is contained, thereby greatly contributing to the development of global economy and the creation of jobs.

In addition, according to the present invention, it is possible to produce advanced composite materials for information technologies (ITs), electronics, semiconductor, LCDs, LEDs and 3D technologies using the advanced multifunctional composite material in which mineral somatid having superior dispersibility is contained, thereby greatly contributing to the development of global economy and the creation of jobs.

Furthermore, according to the present invention, it is possible to produce fibers, clothes, chemicals and polymeric materials using the advanced multifunctional composite material in which mineral somatid having superior dispersibility is contained, thereby providing clothes that can be conveniently used.

## DESCRIPTION OF DRAWINGS

FIG. 1 is a flowchart showing a method of extracting mineral somatid according to an exemplary embodiment of the present invention;

FIG. 2 to FIG. 4 are views showing the effect of mineral somatid on the activity of tumor cells and the proliferation of activated immune cells according to an exemplary embodiment of the present invention;

FIG. 5 shows pictures of the healing progress condition regarding to the wound healing effect experiment of mineral somatid according to an exemplary embodiment of the present invention;

FIG. 6 is a graph indicating a wound healing progress condition of a dermal injury of the mineral somatid according to an exemplary embodiment of the present invention;

FIG. 7 shows pictures of the healing progress condition by a dermal injury experiment of the mineral somatid according to an exemplary embodiment of the present invention;

FIG. 8 is a graph showing a wound healing experiment on a dermal injury using the mineral somatid according to an exemplary embodiment of the present invention;

FIG. 9 is a graph showing a weight comparison analysis of the mineral somatid according to an exemplary embodiment of the present invention;

FIG. 10 shows pictures of samples treated for arthritis healing including the mineral somatid according to an exemplary embodiment of the present invention;

FIG. 11 shows pictures of the arthritis severity progress using the mineral somatid according to an exemplary embodiment of the present invention;

FIG. 12 is a table presenting the degree to which arthritis is relieved using the mineral somatid according to an exemplary embodiment of the present invention;

FIG. 13 and FIG. 14 are a graph and a table showing measurements of the mineral somatid according to an exemplary embodiment of the present invention related to inflammatory cytokine;

FIG. 15 is a table presenting the effect that the mineral somatid according to an exemplary embodiment of the present invention has on the promotion of growth;

FIG. 16 and FIG. 17 are graphs showing the measurement results of antigen-specific antibodies that the mineral somatid according to an exemplary embodiment of the present invention has in serum;

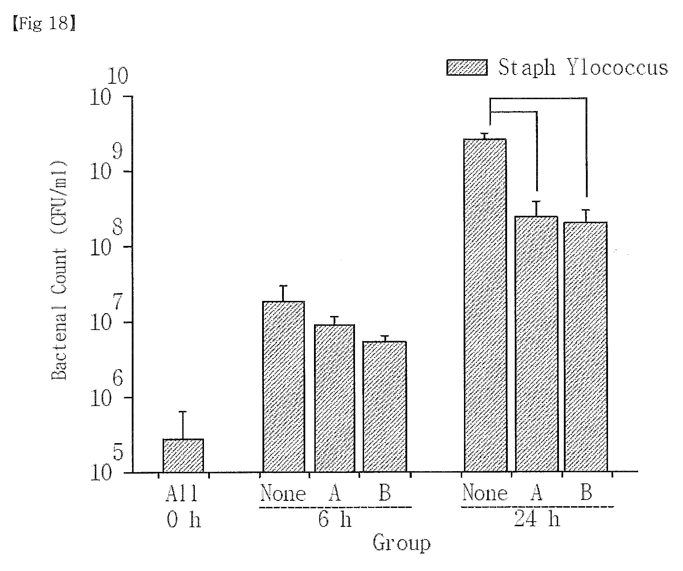

FIG. 18 is a graph showing the measurement results of antibacterial effect to multidrug resistant pathogens (super bacteria) that the mineral somatid according to an exemplary embodiment of the present invention has;

FIG. 19 shows a picture of the initial state in an anti-oxidation effect experiment using the mineral somatid according to an exemplary embodiment of the present invention;

FIG. 20 and FIG. 21 show pictures of the results of the anti-oxidation effect experiment using the mineral somatid according to an exemplary embodiment of the present invention;

FIG. 22 to FIG. 24 are vies showing the results of VOC deodorization and ammonia deodorization using the mineral somatid according to an exemplary embodiment of the present invention;

FIG. 25 and FIG. 26 are views showing a panel containing mineral somatid which is used for an experiment of measuring the electromagnetic radiation absorption and shielding ability of the mineral somatid according to an exemplary embodiment of the present invention;

FIG. 27 to FIG. 29 are views showing the results of the experiment of measuring the electromagnetic radiation absorption and shielding ability of the mineral somatid according to an exemplary embodiment of the present invention;

FIG. 30 is a view showing the results of an experiment of measuring the specific absorption rate of the mineral somatid according to an exemplary embodiment of the present invention;

FIG. 31 is a view showing the results of an experiment of measuring the heat-generating ability of the mineral somatid according to an exemplary embodiment of the present invention;

FIG. 32 to FIG. 34 are views showing the results of an experiment of determining whether or not electricity is charged by the mineral somatid according to an exemplary embodiment of the present invention; and

FIG. 35 to FIG. 37 are views showing the investigation and analysis results of mineral somatid QELBs and microbial species which are re-cultured in the method of FIG. 3 according to an embodiment of the present invention.

**BEST MODE**

Reference will now be made in detail to exemplary embodiments of the present invention with reference to the accompanying drawings. Throughout this document, reference should be made to the drawings, in which the same reference numerals and signs are used throughout the different drawings to designate the same or similar components. In the following description of the present invention, detailed descriptions of known functions and components incorporated herein will be omitted when they may make the subject matter of the present invention unclear.

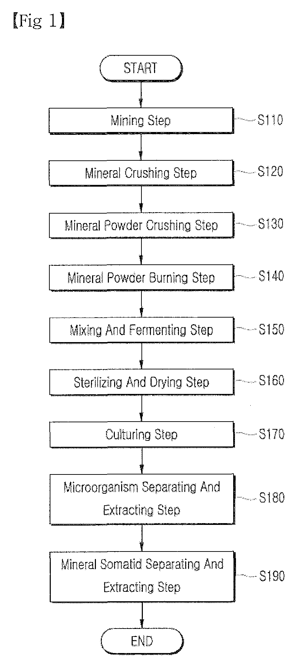

FIG. 1 is a flowchart showing a method of extracting mineral somatid according to an exemplary embodiment of the present invention.

A process shown in FIG. 1 is carried out in order to mass-produce an advanced multifunctional composite material according to the present invention more easily and at a low cost. The advanced multifunctional composite material is produced by combining QELBs (mineral somatid) that show active signs of life and have multiple functions, such as healing, with a ceramic powder made of various kinds of microbial materials which can persist when heated at a high temperature of 1,000°C for 10 hours.

A natural mineral mining step is carried out by mining a natural mineral from the earth (S110). The natural mineral contains inorganic matters, such as $SiO_2$, $Al_2O_3$, $Fe_2O_3$ and $MgO_3$, is not contaminated, and does not have heavy metals or a radioactive substance harmful to living bodies.

A mineral crushing step is carried out by crushing the natural mineral into powder consisting of particles, the size of which ranges from 320 meshes to 2 nanometers (S120). Here, the natural mineral contains a large amount of inorganic matters, such as $SiO_2$, $Al_2O_3$, $Fe_2O_3$ and $MgO_3$.

When the mineral powder crushing step is completed, a mineral powder refining step is carried out by refining the mineral powder by removing heavy metals and radioactive substances harmful to living bodies from the mineral powder (S130).

Then, a mineral powder burning step is carried out by burning the refined mineral powder in order to change the mass, specific gravity, number of electrons and ion number of the refined mineral powder (S140).

The mineral powder burning step includes a first burning process of calcining the mineral powder by heating the mineral powder at a temperature ranging from 50 to 300°C for 2 to 3 hours, and after the completion of the first burning process, a second burning process of burning the mineral powder a second time by heating the mineral powder at a temperature ranging from 300 to 850°C for 30 minutes to 10 hours.

Afterwards, a mixing and fermenting step is carried out by producing a mineral mixture powder by mixing certain ratios of $H_2O$ and a natural plant extract, more particularly, an agar extract, into the burned mineral powder and then activating the mineral somatid and microorganisms by fermenting the mineral mixture powder for a certain time (at S150).

The mixing and fermentation step mixes 70% the mineral powder (crushed at a size of 320 meshes), 25% $H_2O$ and 5% the natural plant extract (obtained from roots, stems or leaves of agar), putting the mixture into a container, and then ripening and fermenting the mixture in a ripening chamber at a temperature ranging from -10 to 200°C for 10 to 90 days so that the mineral somatid and microorganisms are activated.

At this time, the mineral powder is crushed to the size of 320 meshes or smaller, so that the heavy metals and harmful components are removed from the crushed mineral powder that is used.

Afterwards, a sterilizing and drying step is carried out by rotating the mineral powder mixture, which has been ripened and fermented, inside a calciner at a high temperature of 180°C or below for 30 minutes to 2 hours and then drying the resultant powder at a temperature ranging from 150 to 200°C using a drier (S160).

After that, a ceramic powder mixture culturing step is carried out (S170). This step includes putting the dried mineral powder mixture into a container, adding a solution to which a certain amount of water, distilled water, magnetized water, glucose or sugar is added to the mineral powder mixture, adding a nutrient medium designed to support the growth of microorganisms to the mineral powder mixture to which the solution is added, and culturing the mineral powder mixture in the resultant powder mixture for at least one hour.

Here, conditions for culturing the mineral powder mixture according to the present invention include a culture medium, such as yeast extraction mineral medium (YEM), tryptic soy broth (TSB) medium, M9 medium or Luria broth (LB) medium. Culturing temperature ranges from 30 to 37°C.

In addition, the composition of YEM in the medium consists of $Na_2HPO_4 \cdot 12H_2O$ 3.5g, $K_2HPO_4$ 1.0g, $MgSO_4 \cdot 7H_2O$ 0.03g, $NH_4Cl$ 0.5g, yeast extract 4.0g, agar 15.0g, and distilled water 1.0L.

Afterwards, a microorganism extraction step of extracting various kinds of microorganisms including the mineral somatid (QELBs) from the ceramic powder mixture is carried out (S180). The mineral somatid continues life activity and radiates energy after the ceramic powder mixture containing the mineral somatid is sterilized at a high temperature of 1,000°C or higher for at least 10 hours in an autoclave.

In addition, a step of extracting the mineral somatid from the extracted microorganisms is carried out (S190).

According to the present invention as set forth above, it is possible to manufacture an advanced multifunctional material using mineral somatid that is extracted through the above-described steps S110 to S190. The produced advanced multifunctional material has multiple functions, including cancer suppression, immune enhancement, skin reconstruction, self-heating, cold activeness, VOC deodorization and harmful electromagnetic radiation shielding and absorption.

That is, the present invention manufactures an advanced ceramic material that can be used in a variety of industrial fields by further performing a step of manufacturing an advanced multifunctional material by mixing certain amounts of mineral somatid and $H_2O$. The advanced multifunctional material is manufactured by mixing, by weight, 65% mineral somatid, 15% ceramic powder including $SiO_2$, $Al_2O_3$, $Fe_2O_3$ and $MgO_3$, and 20% $H_2O$.

The following experiments were performed in order to evaluate the functions and effects of mineral somatid that is extracted by the above-described method.

**[Experiment 1]**

Effects of Mineral Somatid on Activation of Tumor Cells, such as Colon Cancer Cells and Gastric cancer Cells and on Proliferation of Activated Immune Cells

In order to examine the effects of mineral somatid according to the present invention on the activation of tumor cells using a human tumor cell line and confirm the effects of mineral somatid according to the present invention on the activated state of immune cells, the following experiment was performed.

1. Preparation of Cancer Cell and Experimental group

The activated cell state in a group to which mineral somatid was added and a control group to which Ge was added were analyzed using the following cancer cell lines.

1) Caner cell lines

- SNU1: Human gastric cancer (human gastric cancer cell line)
- SNUC2A: Human colon cancer (human colon cancer cell line)

For experimental matters, a mineral somatid product, a Ge gel product serving as a powdered limestone control group, and a sample in which mineral somatid related to quantum energy and sun plant were mixed at a 1:1 ratio were used.

Table 1

| Experimental groups for Antitumor Activity of Mineral Somatid |
|---|
| Non-treatment group |
| Mineral somatid treatment group |
| Ge treatment group |
| Mineral somatid + sun plant treatment group |

2. Cancer Cell Culture

Tumor cell lines to be used in the experiment were inputted into a suitable culture fluid (RPMI 1640, 10% FBS; GIBCO Inc., USA) and were maintained in a culture condition of $CO_2$ 5%. The mineral somatid gel and the Ge gel as a powdered limestone control group were divided into cell plates which were cultured following the experimental plan according to concentration. In this case, cells were cultured in 96-well plates by determining their concentrations depending on the cell lines. The cells were cultured for 3 days, during which an increase or decrease in the number of cancer cells was confirmed by a cell number analysis and a livability analysis. The livability of a group was quantified by comparison to the non-treated control group after setting the non-treated control group to 100% by measuring the $OD_{450}$ value using a CCK-8 assay kit (Dojindo Molecular Technologies, Inc., USA).

The SNUC2A cells having a concentration of $3.3X10^4$ cells/ml were divided to 96 well plates at a ratio of 100 $\mu$l/well, and were then treated with mineral somatid and Ge at a concentration of 0.25 mg/ml per well.

The SNU1 cells having a concentration of $1X10^5$ cells/ml were divided to 96 well plates at a ratio of 100 $\mu$l/well, and then were treated with mineral somatid and Ge at a concentration of 1.0 mg/ml per well.

In this experiment, in addition to the mineral somatid and Ge samples, a sample in which sun plant was mixed to mineral somatid at a 1:1 ratio was used.

3. Culture and Activation of Normal Immune Cells

For normal immune cells, spleen cells were extracted from BALB/c mice that were about 20-weeks old (Central Lab. Animal Inc., the Republic of Korea), and were cultured to the maximum level under culturing conditions of RPMI 1640, 37°C and $CO_2$ 5%.

The spleen cells having a concentration of $2*10^6$ cells/ml were treated with Concanavalin A (ConA; Sigma Chemical Co., USA), an agent that induces cell activity, having a concentration of 2.5ug/ml, and were cultured for 3 days. The cell number was measured every 24 hours, and livability was produced by measuring absorbance using CCK-8 assay kit as in the cancer cells, setting the livability of the non-treated control group to 100%, and then comparing the measurement with that of the non-treated control group.

The average and standard deviation were produced by triplicating each experiment, and experiments were repeated several times as required.

The results of the study that followed the experimental condition as stated above are presented as follows.

1. Effects on Tumor Cells and Activated Immune Cells

1) Analysis of Tumor Cell Growth

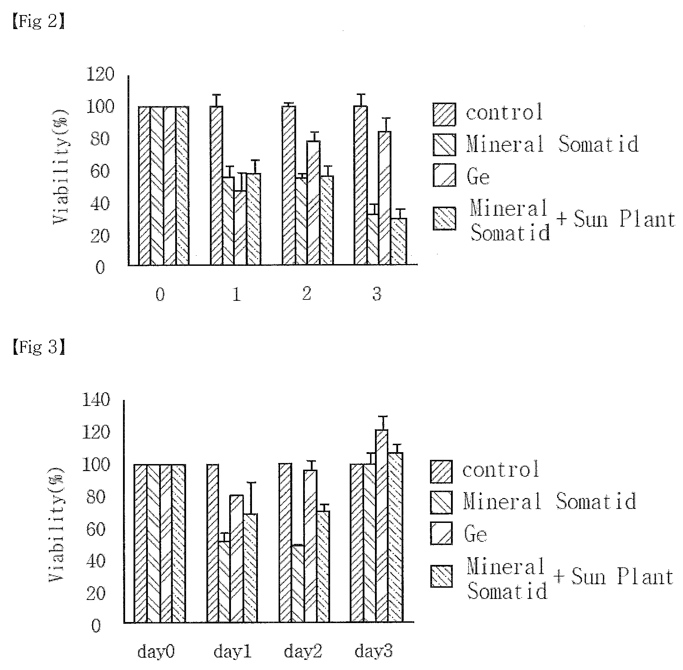

-   In colon cancer cells SNUC2A, when livability was measured every 24 hours for 3 days after the culture of tumor cells, it was observed that livability significantly decreased in the mineral somatid treatment group than in the Ge treatment group at 48 hours and 72 hours (FIG. 2).
-   In gastric cancer cells SNU1, when livability was measured from each experimental group every 24 hours for 3 days, it was observed that livability significantly decreased in the mineral somatid treatment group than in the Ge treatment group at 48 hours and 72 hours (FIG. 3).

2. Effects on Proliferation of Activated Immune Cells

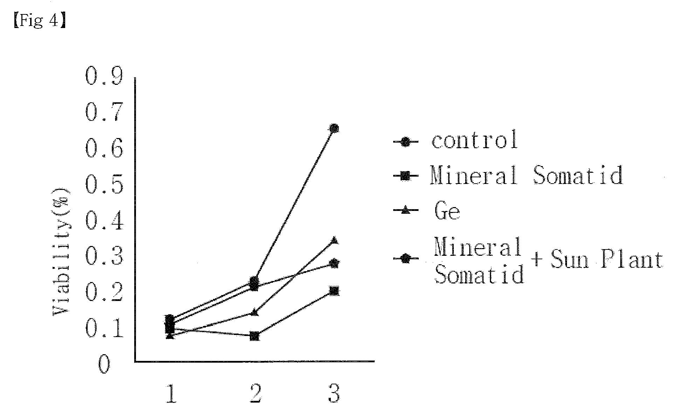

Extracted spleen cells were treated with a sample such as mineral somatid having a concentration of 0.25mg/ml, and the number of living cells was measured 70 hours after culture. The number of cells of the mineral somatid treatment group significantly decreased than that of the Ge treatment group (FIG. 4).

3. Conclusion

The growth of tumor cells was significantly suppressed in the mineral somatid treatment group compared to the non-treatment group and the Ge treatment group. It is concluded that mineral somatid has the effect of suppressing the activity and proliferation of cancer cells. In this experiment, the same effects were not produced by Ge.

Although the effects of mineral somatid on the proliferation of anti-tumor cells were observed from both colon cancer and gastric cancer, the effects appeared at different times. The effects on colon cancer cells became significant on days 2 and 3 after treatment, and the effects on gastric cancer cells became significant on days 1 and 2 after treatment.

[Experiment 2]

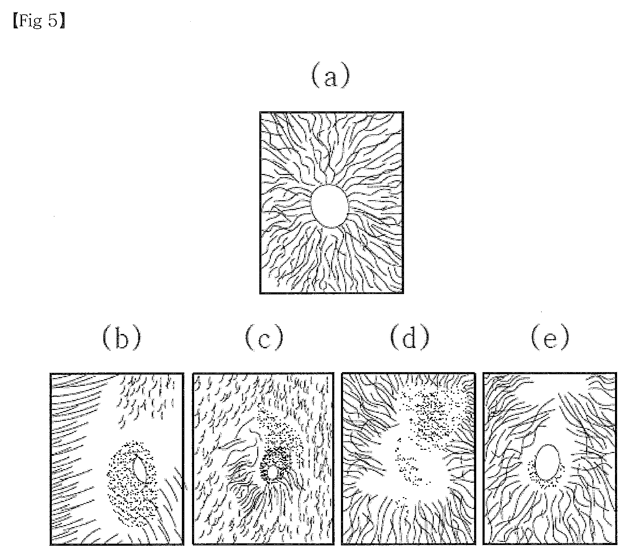

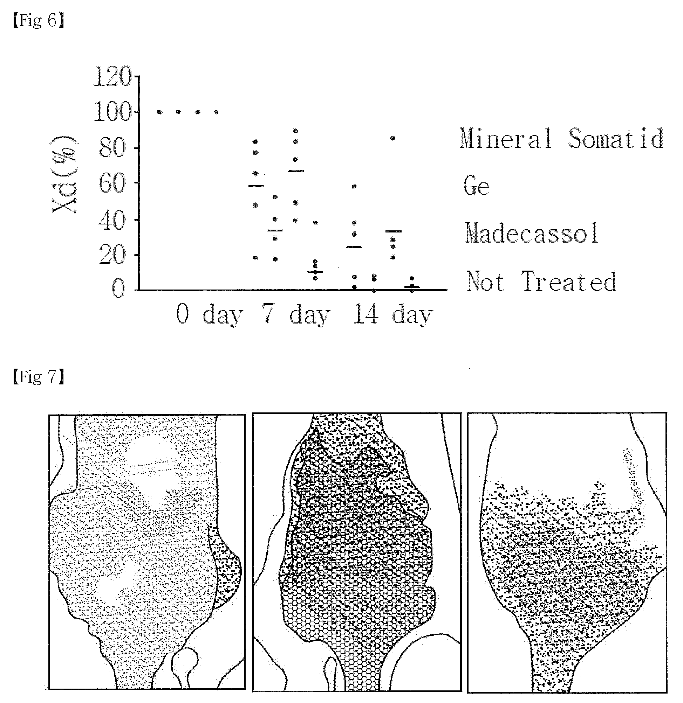

FIG. 5 shows pictures of the healing progress condition regarding to the wound healing effect experiment of the mineral somatid according to an exemplary embodiment of the present invention, and FIG. 6 is a graph indicating a wound healing progress condition of a dermal injury of the mineral somatid according to an exemplary embodiment of the present invention.

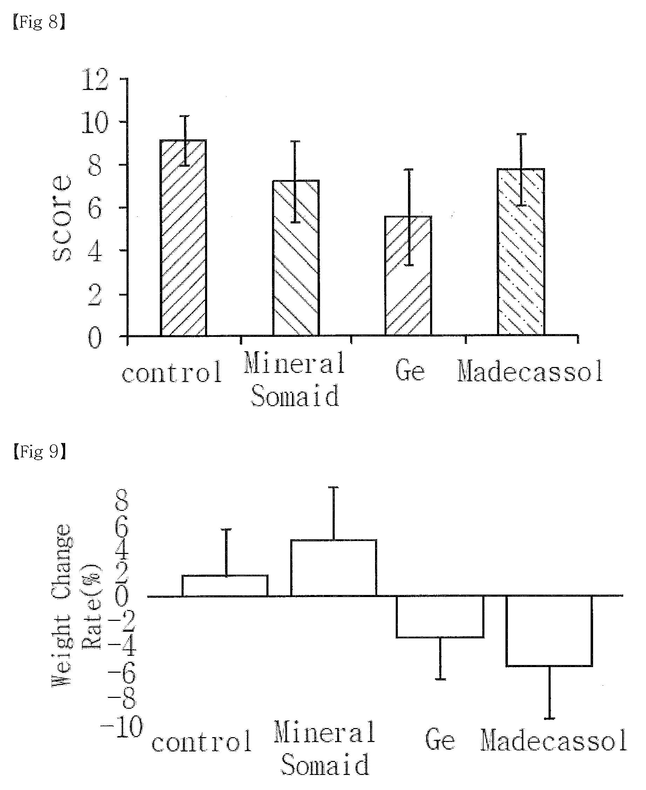

Also, FIG. 7 shows pictures of the healing progress condition by a dermal injury experiment of the mineral somatid according to an exemplary embodiment of the present invention, FIG. 8 is a graph showing the wound healing experiment using the mineral somatid according to an exemplary embodiment of the present invention when the skin is damaged, and FIG. 9 is a graph showing a weight comparison analysis on a dermal injury using the mineral somatid according to an exemplary embodiment of the present invention.

As indicated, the wound healing effect was compared and analyzed using an animal model (mice) to study the effect that influences on dermal regeneration from a dermal injury by a wound using the mineral somatid according to the present invention.

1. Experimental Animal

Purchased 6-week old BALB/c mice were used in the present experiment after one week of acclimatization. The groups that were cross-checked in the experiment are as follows (Table 2). The positive treatments for wound healing, used here, were frequent topical that are currently circulating in the market for the same purpose (Madecassol®; Dong-Gook Pharmaceutical Company, South Korea), and Ge gel was used as a powdered limestone control group.

Madecassol, already on the market, a positive treatment in the experiment, was a substance that was already sanctioned by the authorities to be commercialized. It is known as a treatment including an antibiotic that reduces the inflammation. The main function is acting as a titrated extract of Centella asiatica (TECA), the main component, on the process of formation of new connective tissue so that it can help to form granulation tissues having superior quality. It is known for ensuring that there is no scarring by inducing the normal process of formation of collagen fibers.

Table 2

| Treatment Group of Wound Healing Effect of Mineral Somatid | | |
|---|---|---|
| | Dermal injury experiment | Skin injury experiment |
| Commercial product (Madecassol) treatment group | 5 mice | 5 mice |
| Mineral somatid treatment group | 5 mice | 5 mice |
| Germanium (Ge) treatment group | 5 mice | 5 mice |
| Non-treatment group | 5 mice | 5 mice |

2. Wound Induction and Sample Application Method

Each mouse was isolated in a cage to prevent from affecting the experimental results by leaking an induced wound each other. As a pretreatment, a body region was shaved using an electric shaver in order to induce a wound in that region. Shaving cream was then put on the region to remove the hair completely.

In the dermal injury experiment, after a mouse was put to anesthesia by ether, the epidermis and dermis were removed by using punch, and the wound was induced into a velamentous membrane. Meanwhile, in the skin injury experiment, by removing the outer skin layer with sandpaper after the mouse was put to anethesia by ether, a skin injury was induced. The mineral somatid and Ge gel medicine were treated enough to cover the wound, and a commercial product was used following the manufacturer's instruction.

On the 7th and 14th days after the skin injury was induced, a comparison of the wound healing effect was performed by measuring the wounded region with Vernier calipers after treating the wound with phosphate buffered saline (PBS) and removing the gel medicine with Kimwipes. This process was equally carried out on the non-treatment group and the commercial product treatment group in order to remove variables among the population.

The skin wound was quantified by scoring (or evaluating) the healing progress by comparing the individual wound degree on the sixth day with the individual wound degree on the first day. Wound healing scoring items were determined as follows:

    1. Bursting degree of flare
    2. Concentration of crust
    3. Degree of reduction in the crust size

According to the degree of healing, each item was scored on a scale from 1-5 by its degree. By setting the (evaluation) score of a certain object of non-treatment group as 3 and setting it as a standard, after scoring by comparing each individual from each group, the individuals were measured by adding the scores from all items. The changes in weight of the experimental animals were measured to study the influence of stress that the animals experienced through the medical treatment.

The results of the study that followed the above-stated experimental condition are as follows:

1. Dermal injury experiment

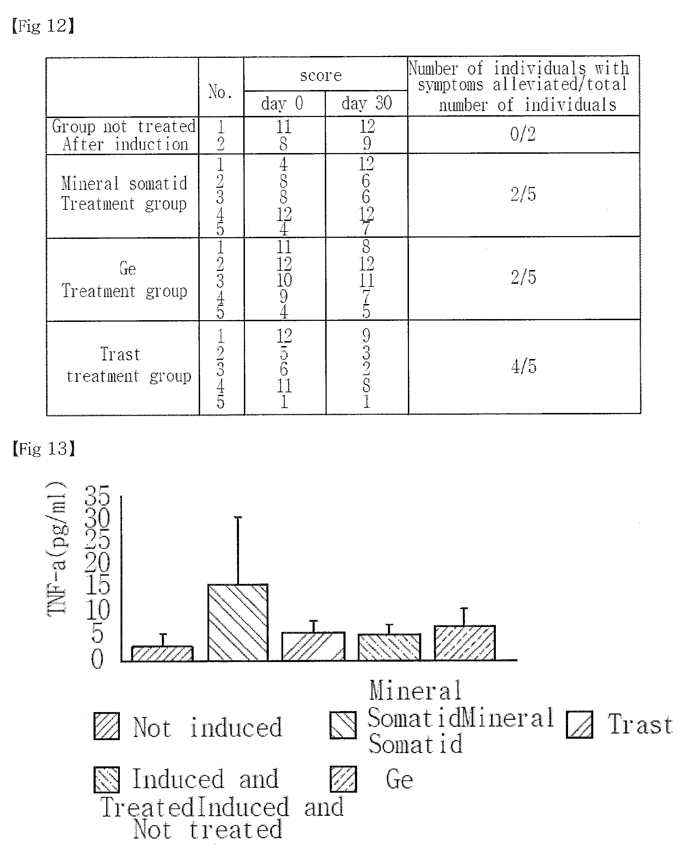

By progressing the dermal injury experiment using a punch, an aspect of the injured region shortly after the dermal damage induction (day 0) and on the experiment end date (day 14) are the same as shown in FIG. 12.

(a) in FIG. 5 indicates the state shortly after the dermal injury induction (day 0), (b) in FIG. 5 indicates the non-treatment group (day 14) shortly after the experiment ended, (c) in FIG. 5 indicates the treatment group shortly after the dermal injury experiment ended (day 14) when the mineral somatid according to the present invention was used. (d) in FIG. 5 indicates the Ge treatment group shortly after the dermal injury experiment end (day 14), (e) in FIG. 5 indicates the Madecassol treatment group shortly after the dermal injury experiment ended (day 14).

In the case of the dermal injury experiment, because the gap among the individuals is large, a significant difference among the groups are not shown. However, the wound recovery speed is fastest in the non-treatment group followed by the Ge group, the mineral somatid group, and finally the Madecassol treatment. The wound residual rate (Xd) is indicated as a percentage by dividing the initial wound size (S0) by the final wound size (Sd), and its equation is as follows.

Increasing the Xd value indicates that the wound recovery is delayed.

$$[\text{Formula 1}]$$

$$\text{Xd}\,(\%) = \frac{Sd}{S0} \times 100$$

2-1. Skin injury experiment

The skin injury experiment was performing by treating a sample after damaging the epidermis of the sample with sandpaper, as shown in FIG. 7. Part (a) in FIG. 7 shows a state shortly after the treatment of the non-treatment group. Part (b) in FIG. 7 shows a state shortly after the treatment of the mineral somatid treatment group according to the present invention. Part (c) in FIG. 7 shows a state shortly after the treatment of the Madecassol treatment group.

In the case of a skin injury, in particular, as indicated in FIG. 8, the wound recovery is fastest in the non-treatment group followed by the Madecassol group, the mineral somatid group, and finally the Ge group. In the average value of each treatment group, a comparison between only the non-treatment group and the Ge treatment group indicated a significant difference.

2-2. Comparative analysis of weight

A weight comparative analysis is shown in FIG. 9. The weight change was compared for each experimental group by measuring the weight shortly after wound induction (day 0) and on the final day of the experiment (day 7) (FIG. 9). The weight increase in the mineral somatid treatment group was significantly related to the difference between the mineral somatid treatment group and the Madecassol or Ge treatment group. In addition, the difference between the Madecassol treatment group and the non-treatment group is significant.

3. Conclusion

The process of normal wound recovery had the complicated progress including: inflammation in which a blood platelet coagulation occurs and cytokine needed for the wound to recover is released; granulation which synthesizes a new extracellular matrix by moving fibroblasts to a wound; epithelialization, which is a state of thickening the epidermis and increasing the size of the basal cells; fibroplasia, which is a state of forming the internal structure of a wound by collagen fibers; and finally, the contraction of the wound.

In a dermal injury, the non-treatment group (free healing group) is shown to be most effective, followed by the Ge group, the mineral somatid group according to the present invention, and the Madecassol group.

Madecassol, because of a function the main component, is less effective in terms of the wound healing of the dermis. Consequently, the significance of this experiment is that the mineral somatid is different from germanium (Ge) in that its effects are similar in degree to those of Madecassol in terms of the wound recovery of the dermal stratum. This tendency is also shown in the epidermal cells.

In the other experiment in which an epidermal wound by abrasion was carried out, only the Ge treatment group added as a control group was measured to be significantly lower than the other groups. In addition, the mineral somatid according to the present invention has a similar effect to that of the non-treatment (free healing) or Madecassol since the mineral somatid group according to the present invention did not have a significant difference with non-treatment group (free healing group) and Madecassol treatment group.

In addition, the mineral somatid is a gel product of which the healing effect on the skin injury is concluded to be similar to that of Madecassol, considering that the crust covering the wound, which was formed by PBS, became tender during the gel removing process for photography. However, the recovery of the that non-treatment group faster than that of the commercial product treatment group is an unexpected result, and it is unclear whether or not this result is caused by the difference in the animal cells or animal species.

On the other hand, as a result of measuring the weight change to study the stress level regarding an inflammatory response, the weight increase in the mineral somatid treatment group showed a significant difference compared to the weight decrease in the Madecassol or Ge treatment group, and showed a larger weight increase compared to the non-treatment control group, but the difference was not significant. Considering the weight increase tendency similar to that of the non-treatment group, a negative effect, such as a decrease in the weight of animals that can be seen in an antibiotic treatment, was not observed from the mineral somatid, but the possibility of a general effect of increasing the weight was indicated.

By the results stated above, the mineral somatid indicated that its wound healing recovery properties are the same or more effective compared to commercial products. The mineral somatid was more effective than Madecassol in dermal injuries, was similarly effective to Madecassol in epidermal injuries, and showed a similar rate of weight increase as the non-treatment group differently from the Ge or Madecassol treatment group. It is concluded that the mineral somatid has a similar level of wound healing effects as a commercial product without any side effects.

**[Experiment 3]**

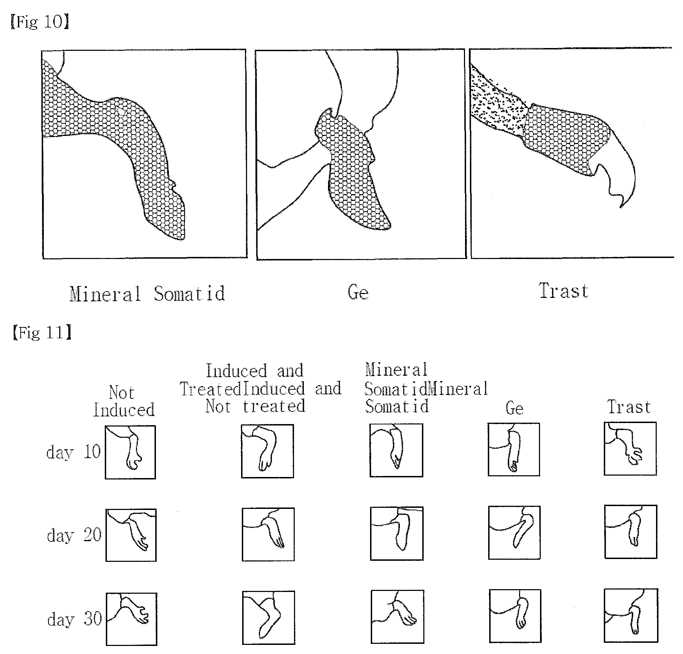

FIG. 10 shows pictures of samples treated for healing arthritis using the mineral somatid according to an exemplary embodiment of the present invention, FIG. 11 shows pictures of the arthritis severity progress using the mineral somatid according to an exemplary embodiment of the present invention, and FIG. 12 is a table presenting the degree to which arthritis is relieved using the mineral somatid according to an exemplary embodiment of the present invention.

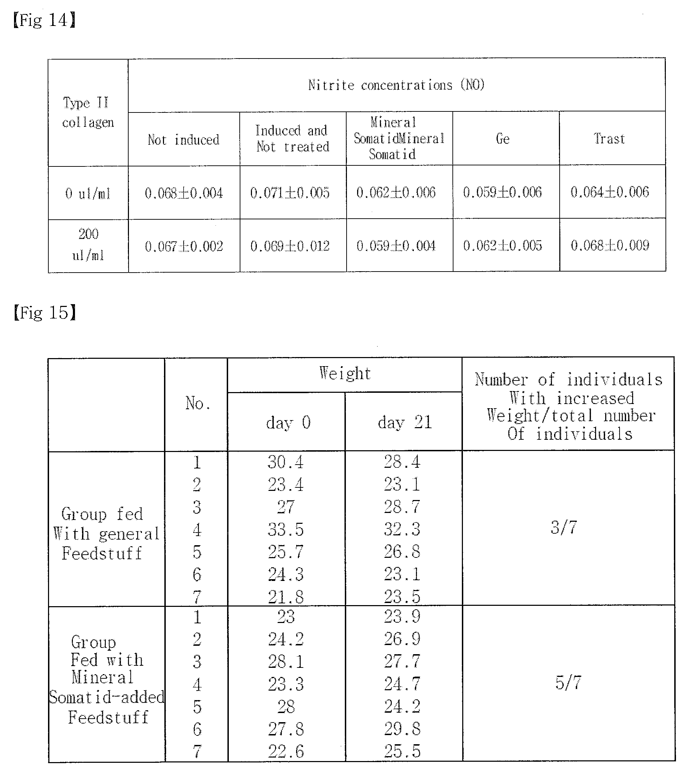

In addition, FIG. 13 and FIG. 14 are a graph and a table showing measurements of the mineral somatid according to an exemplary embodiment of the present invention related to inflammatory cytokine.

As shown in the figures, the influence of mineral somatid on arthritis healing was examined by comparing and analyzing the arthritis healing effects in animal models using mice. In order to perform a more reliable analysis, the concentration in blood of TNF-$\alpha$, or an inflammatory cytokine, and nitric oxide were measured.

1. Experimental Animal

Purchased 6-week old BALB/c mice (Central Lab. Animal Inc., the Republic of Korea) were used in the present experiment after one week of acclimatization. Groups which were compared and analyzed in the experiment were set as in Table 3. As a positive sample for arthritis healing, a commercially available patch type healer (Trast®; SK Chemical, the Republic of Korea), which is widely distributed for the same purpose in the market, was used. In addition, a powdered limestone control group was treated with a Ge gel.

The commercially available Trast patch, which was used as the positive sample in the experiment, contains piroxicam as its major component, which is known to have antiphlogistic, pain relief and antipyretic effects.

Referring to the specific mechanism of piroxicam, it is known that piroxicam obstructs the synthesis of prostaglandin that causes inflammation and pain, acts on thermoregulatory center of the hypothalamus to have an antipyretic effect, and suppresses the sensitivity of pain receptors to have a pain relieving effect. Piroxicam is also considered to have antiinflammatory activity based on the anti-activity mechanism of neutrophils.

Table 3

| Experimental Groups for Arthritis Heating Effect of Mineral Somatid | | |
| --- | --- | --- |
| Arthritis induced | Commercial product (Trast) treatment group | 5 mice |

(continued)

| Experimental Groups for Arthritis Heating Effect of Mineral Somatid | | |
|---|---|---|
| | mineral somatid treatment group | 5 mice |
| | Ge treatment group | 5 mice |
| | non-treatment group | 2 mice |
| Arthritis not induced | non-treatment group | 2 mice |

2. Arthritis Induction and Sample Application Method

In order to induce arthritis to experimental animals, type 2 collagen (Chondrex Inc., USA) was dissolved into 0.05M of acetic acid at a concentration of 2mg/ml. Complete Freund's adjuvant (CFA; Sigma Chemical Co., USA) was prepared, and the CFA and the type 2 collagen solution were mixed at a 1:1 ratio (v/v) so as to be emulsified. The mixture solution was injected with $100\mu$g to the tail of each individual. At 2 weeks after the first inoculation, a second inoculation was performed for the purpose of boosting. Type 2 collagen and incomplete Freund's adjuvant (IFA; Sigma Chemical Co., USA) were mixed at a 1:1 ratio (v/v), and the mixture solution was injected by $100\mu$g to the tail of each individual.

Arthritis appeared between 4 to 6 weeks after the first injection of the mixture solution of type 2 collagen and CFA. At the seventh week, the mineral somatid and the Ge gel medicine were used enough to cover the joint once a day, and the commercial product was properly used by following the manufacturer's instructions.

3. Arthritis Severity Analysis

The degree of expression of arthritis was observed once a week until the third week and twice a week thereafter, and was visually estimated. The degree of edema was estimated by photographing the mice on days 10, 20 and 30 by compensation after the application of samples.

For objective measurement, arthritis severity scores for all 4 feet were evaluated from 0 to 3 points, with 12 points being the highest for each individual. For each individual, arthritis symptoms on day 0 and the degrees of symptoms on day 30 were quantified as blind scoring. Afterwards, the number of individuals whose symptoms were alleviated with respect to the total number of each group was indicated, and the results were compared.

Figure 1. Reference for scoring of arthritis severity

a. score 0: Normal front/rear leg, neither flare nor edema observed

b. score 1: Flare and edema partially observed

c. score 2: Flare and edema considerably observed, but no ankylosis observed

d. score 3: Severe flare and edema observed, and

ankylosis observed

4. Measurement of Tumor Necrosis Factor- $\alpha$ (TNF-$\alpha$) Concentration

At the final day (day 30) after treatment, blood was extracted from the heart of the mice, and serum was separated from the blood. In the separated serum, the concentration of TNF-$\alpha$, inflammatory cytokine, was measured using TNF-$\alpha$ ELISA kit (eBioscience Inc., USA) in which a single clone antibody was used following the manufacturer's instructions.

5. Measurement of Nitric Oxide (NO)

Spleen cells were extracted and divided into 96 well plates by $5*10^3$ cells per each well, and were stabilized in RPMI 1640 (10% FBS, 1% antibiotics; GIBCO Inc., USA) at 37°C under the culture condition of $CO_2$ 5% for 24 hours. Afterwards, type 2 collagen was treated at concentrations of $0\mu l/ml$ and $200\mu l/ml$. After 48 hours, the supernatant was collected, and a measurement was performed using a total NO assay kit (R&D systems, USA) following the manufacturer's instructions.

The results of the study that followed the experimental conditions stated above are presented as follows.

1. Arthritis Severity Progress

When samples were treated as shown in FIG. 10 after the arthritis was induced with type 2 collagen, results shown in FIG. 11 occurred.

FIG. 11 shows the degree of edema in the rear legs of each experimental group on days 10, 20 and 30 after treatment. The severity was quantified by scoring on the first day of experiment (day 0) and the final day of experiment (day 30) in order to examine the degree of arthritis healing. The results are presented in FIG. 12.

2. Appearance of TNF-$\alpha$

The quantity of TNF-$\alpha$ appearing in individual experimental animals on the final day of experiment (day 30) was compared according to each experimental group (FIG. 11). The quantity of TNF-$\alpha$ decreased in the mineral somatid treatment group, the Ge treatment group and the Trast treatment group more than in the group which was not treated after arthritis was induced, but any significant difference among the mineral somatid treatment group, the Ge treatment group and the Trast treatment group did not appear. All of the three groups appeared to have similar levels to the control group in which inflammation was not induced.

3. Concentration of Nitric Oxide (NO)

On the final day (day 30) of the experiment, the concentration of NO secretion from the spleen cells of each experimental group was measured, and the results were compared (FIG. 12). The NO concentration was lower in the mineral somatid treatment group, the Ge treatment group and the Trast treatment group than in the non-treatment group, and the NO concentration of the mineral somatid treatment group was similar to that of the Trast treatment group. However, any significant difference did not appear when the mineral somatid treatment group was compared with the Ge treatment group or the powdered limestone control group.

4. Results

While the origin of rheumatoid arthritis has not been clarified, one important characteristic of the immune reaction in rheumatoid arthritis is the imbalance of a variety of inflammatory cytokines (TNF-$\alpha$, IL-1$\beta$) and anti inflammatory cytokines (TGF-$\beta$) secreted from lymphocytes related to rheumatoid arthritis, macrophage, or the like. Nitric oxide (NO), another etiological factor of rheumatoid arthritis, is known to lead to the malfunction of T cells in systemic lupus erythematosus, an autoimmune disease, and rheumatoid arthritis.

It is known that type 2 collagen can cause arthritis that is very similar to rheumatoid arthritis to animals, and the collagen induced arthritis (CIA) model using the same is an example. This experiment used the CIA model in order to examine the clinical course of arthritis and changes in inflammatory mediators, such as TNF-$\alpha$ or NO, in response to mineral somatid treatment.

The clinical progress of arthritis was visually measured based on the overall variation and the degree of joint edema, and the results of the respective groups were compared with each other. In the control group that was not treated after arthritis was induced, arthritis symptoms slightly worsened without being alleviated with the passage of time. In contrast, as expected, Trast, a commercial product that is commercially distributed for the purpose of alleviating arthritis or muscular pain, was proven most effective in alleviating arthritis symptoms. In addition, the mineral somatid treatment group had a rate of symptom relief of about 40%, i.e. symptoms of 2 mice from among the 5 mice were alleviated, compared to the non-treatment group in which no improvement in symptoms was observed.

Next, variations in TNF-$\alpha$ and NO, inflammatory mediators of rheumatoid arthritis, were examined. In each group, the aspects of TNF-$\alpha$ and NO appeared to correlate with each other. This seems reasonable considering that TNF-$\alpha$ and IL-1$\beta$, inflammatory cytokines, are strong stimulating factors of NO overproduction. In the inflammatory mediator experiment, the numerical value of the inflammatory mediator was low across the entire groups due to the insufficient number of the murine spleen cells. Therefore, no significant differences were observed and only slight differences appeared between the groups.

It is notable that the mineral somatid treatment group had a smaller amount of inflammatory material or cytokine than the non-treatment group and its inflammation relieving effect was similar to that of the commercially available product. However, the mineral somatid treatment group did not have a significant difference from the Ge treatment group or the powdered limestone control group.

Considering all the above-mentioned results, although the effect of the mineral somatid on arthritis was not visually significant as in the commercially available product, arthritis symptoms tend to be relieved by the mineral somatid compared to the non-treatment group. Considering specific mechanisms such as the inflammatory mediator, the degree of inflammation relief was similar to that of the commercially available product even though its effect was not significant.

Considering that the major function of Trast is to alleviate symptoms or pain rather than curing rheumatoid arthritis, i.e. allopathic effect of inflammatory reaction and subsequent edema, it is concluded that the mineral somatid has the effect of alleviating the inflammatory reaction at the joint to a certain degree although it does not have the effect in curing edema.

**[Experiment 4]**

FIG. 15 is a table presenting the effect that the mineral somatid according to an exemplary embodiment of the present invention has on the promotion of growth.

As shown in the figure, in order to examine physiological effects of the mineral somatid, the effects of the increased weight of animal models using mice caused by the mineral somatid were studied through comparison and analysis. In order to more accurately judge the influences, changes in the diet organs were also observed when the experiment was completed.

1. Experimental animals and Feeding Method

In order to prevent any interference of nonspecific effects, Ovalbumin TCR-Transgenic mice that were at least 9 week old (Central Lab. Animal Inc., the Republic of Korea) were used in the present experiment after one week of acclimatization. Groups compared and examined in the experiment were set as in Table 4. A control group was fed with general feedstuff for mice, and experimental groups were divided into a group which was fed with feedstuff produced by mixing mineral somatid pills with general feedstuff and a group which was fed with only mineral somatid pills. As for drinking water, the control group was fed with tap water, and the two experimental groups to which the mineral somatid was fed as feedstuff were fed with water that was produced by putting mineral somatid 3g (1 teaspoon) into water 100 ml, boiling the mixture water, and mixing the supernatant with water at a ratio of 1:1 (v/v) (breeding water). In addition, mineral somatid mattresses were laid for the two experimental groups to which the mineral somatid was fed.

Table 4

| Experimental Groups for Growth Promotion Effect of Mineral Somatid | | | |
|---|---|---|---|
| | 1 | 2 | 3 |
| Feedstuff | General feedstuff | Mineral somatid added feedstuff | Mineral somatid pills |
| Drinking water | Tap water | Breeding water | Breeding water |
| Mattress | None | Mineral somatid mattress | Mineral somatid mattress |
| | 7 mice | 7 mice | 7 mice |

The feed results were presented by measuring the weights on day 0 and after 1, 2 and 3 weeks. The mice were dissected on the final day of the experiment after 3 weeks, and the digestive organs were compared with each other.

When the study was performed following the above-stated experimental conditions, the following results were obtained.

As shown in FIG. 15, the weights of individual experimental animals were measured right before the start of the feed experiment (day 0) and on the final day of the experiment (day 21), and the number of mice that showed an increase in the weight was presented with respect to the total number of mice. In the general feedstuff group, only 3 mice from among total 7 mice gained weight. In contrast, in the diet group using the mineral somatid-added feedstuff, 5 mice gained weight. The experimental group, to which only the mineral somatid pills were fed, experienced cannibalism from 3 days after the start of the experiment, and all mice in this group died during 12 days.

In addition, the mice were dissected on the final date of the experiment (day 21). When the digestive organs of the diet group to which the general feedstuff was fed were compared with the digestive organs of the diet group to which the mineral somatid-added feedstuff was added, there were no specific impressions, such as a deformed digestive organ, except for the color of excrements.

According to above-described study results, it can be appreciated that the mineral somatid pills failed to provide sufficient nutrition since cannibalism occurred from 3 days after the start of the experiment in the group when only the mineral somatid pills of the present invention were used. In contrast, in the diet group to which the mineral somatid-added feedstuff was fed, the weight significantly increased than in the group to which the general feedstuff was fed.

Although the mechanism of action is unclear at present, it is possible that the mineral somatid enters living bodies together with the general feedstuff and help the absorption of nutrients in the feedstuff. In addition, since no abnormality in the digestive organs was visually observed in the group to which the mineral somatid was fed together with the general feedstuff, it is concluded that there is no specific toxicity caused by the intake of the mineral somatid.

In other words, it is indicated that the treatment with the mineral somatid according to the present invention suppresses tumor cells that would otherwise proliferate without a balance with the surroundings and suppresses the proliferation of immune cells that were artificially activated such as to induce excessive cell division. It is therefore regarded that the mineral somatid has the function of controlling an immune response. Furthermore, the effect of promoting an increase in the weight can be regarded that the mineral somatid treatment acts to allow living bodies to maintain equilibrium, i.e. to maintain health, which is an interesting result.

Although the mineral somatid treatment failed to obtain the similar level of wound healing effect or arthritis healing effect as a medicine, it showed a certain level of inflammation reducing effect. From this, a sufficiently-positive effect due to long-term application of the mineral somatid is expectable. In particular, several experimental results suggest that the effects of the mineral somatid relate to the homeostasis maintenance of living bodies, thereby achieving its own healing effect. This characteristic suggests that it is necessary for the mineral somatid to be taken or applied for a long time as a kind of supplement in daily life unlike medicines. It is also notable that neither specific digestive disorder nor cytotoxicity was observed following the intake of the mineral somatid into living bodies.

Based on the results of this experiment, it is concluded that the mineral somatid treatment assists in homeostasis and activity. It is also concluded that the mineral somatid acts for the restoration of a living body from a damaged or unbalanced condition into a normal condition and helps a living body be healthy.

**[Experiment 5]**

Experiment 5 is a study about the mechanism of homeostasis maintenance of living bodies, and murine experimental model about allergic response was applied. For this, the suppression effect of the mineral somatid on allergy was analyzed from allergy-induced mice. At the same time, variations in cytokine excreting from cells and Regulatory T ($T_{reg}$) cells were examined.

1. Research Method

1.1. Animals

Experiment was carried out using 8 to 10-week old male or female mice having OVA323-339-specific and I-Ad restricted TCR-$\alpha\beta$ (V$\alpha$3/V$\beta$15) in BALB/c genetic background, imported from Jackson Laboratory (Main, USA). Cloth containing the mineral somatid and common cloth were provided from Quantum Energy Co., Ltd. In this experiment, it was first intended to put the cloth containing the mineral somatid and the common cloth on mice in the form of dress. However, since it was impossible that the cloth maintains the form of dress, the cloth was laid on the bottom of breeding cages instead.

2. Results

2.1. Measurement Results of Antigen-Specific Antibody in Serum

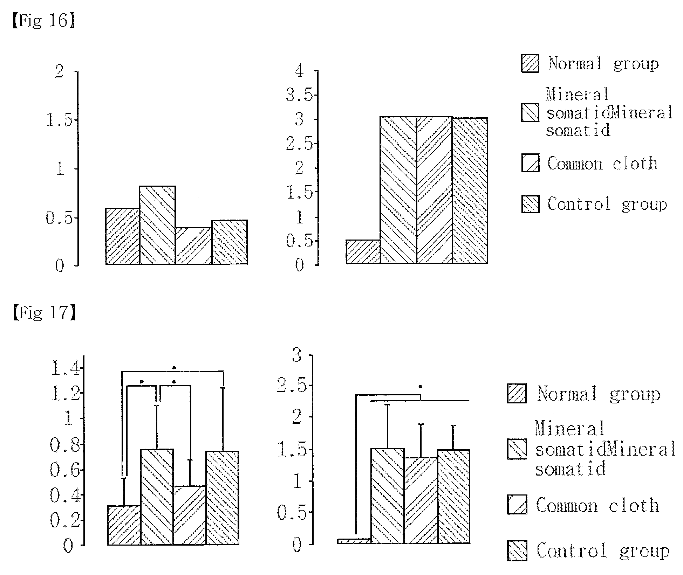

As shown in FIG. 16, total IgE in the graph a) and OVA-specific IgG in the graph b) appeared in the mice serum which were subjected to i,p re-sensitization after OVA was orally administered. When normal groups (groups to which allergy was not induced) and control groups (allergy-induced groups) were compared, all groups (control groups) treated with ovalbumin (OVA) had a great increase in the specific IgG level in the serum, which significantly differs from normal groups.

However, no significant differences appeared between the group treated with cloth containing the mineral somatid, the group treated with common cloth and the non-treatment groups. The group treated with the mineral somatid cloth had higher total IgE than the other normal groups and the control groups, and showed a significant difference from the group treated with common cloth.

In all groups treated with OVA, the specific IgG level of OVA in the serum greatly increased, which significantly differ from the normal groups. However, no significant difference appeared between the experimental groups and the control groups.

In addition, as shown in FIG. 17, total IgE appeared higher in all groups treated with OVA than in the normal groups. In particular, a significant difference appeared between the group treated with cloth containing the mineral somatid and the non-treatment group. In addition, the group treated with cloth containing the mineral somatid showed significantly higher IgE level than the group treated with common cloth.

**[Experiment 6]**

Antibacterial Effect of Mineral Somatid to Multidrug Resistant Pathogens (Super Bacteria)

1. Experimental Method

1) Subject Bacteria

A. Bacterial Species

[0144]

　　∘ *Staphylococcus aureus* N315 (Resistant to 4 sorts of antibiotics)

B. Amount of Inoculation

[0145]

　　∘ Initial Inoculation Concentration: $4.53*10^5$ CFU/ml

2) Mineral Somatid Used in Experiment 6

A. Type

Table 5

| Samples | Pretreatment condition | Remarks |
|---------|------------------------|---------|
| A | 1000°C, 4 H | A certain amount of ash mixed |
| B | 1000°C, 4 H | Estimated to be more heated since positioned at the center of flame |

B. Experimental Medium and Treatment Capacity

[0147]

　　∘ Muller-Hinton Broth (MHB) 4 ml ∘ 1 small spoon (~0.1 grams) per MHB liquid medium 4 ml

3) Method of Measuring Number of Bacteria

◦ Strain to be used for inoculation was streaked in 5% sheep blood agar in a -80°C stock, followed by stationary culture at 37°C for 20 hours.

◦ One colony was selected, was streaked again in 5% sheep blood agar, and then was subjected to stationary culture at 37°C for 20 hours.

◦ One colony was selected, was inoculated in 3 ml MHB, and then was subjected to shaking culture at 37°C for 20 hours.

◦ At the next day, the medium was diluted in MHB at a 1:100 ratio, and 40μl was taken from the medium and inoculated in 4 ml MHB.

◦ 1 small spoon of stone powder A or B was added to experimental groups, and shaking culture was started at 37°C.

◦ 100 μl was taken from each sample at 0, 6 and 24 hours after culture, and was decimally diluted.

◦ 100 μl of the diluted solution was dropped onto and allowed to be absorbed to Muller-Hinton Agar, stationary culture was performed at 37°C for 20 hours, and the amount of bacteria was measured by counting the number of colonies that appeared after the stationary culture.

4) Classification of Experimental Group

Table 6

| Treatment substance<br>Inoculated bacteria | A | B | None |
|---|---|---|---|
| *S. aureus* N315 | Group1 | Group2 | Group3 |
| None | Group7 | Group8 | Group9 |

2. Results

Table 7

| *Staphylococcus aureus* N315 | | | |
|---|---|---|---|
| Culturing time | Treatment method | Number of bacteria | Ratio (%) |
| 6 h | Control group | 1.93E+07 | 100 |
| | A | 8.67E+06 | 45 |
| | B | 5.33E+06 | 28 |
| 24 h | Control group | 2.50E+09 | 100 |
| | A | 2.33E+08 | 9 |
| | B | 2.00E+08 | 8 |

3. Conclusion

As shown in FIG. 16, both of mineral somatid samples A and B were effective in suppressing the growth of *Staphylococcus aureus* N315, multidrug resistant pathogen.

**[Experiment 7]**

Anti-oxidation Effect of Mineral Somatid

1. Experimental Condition

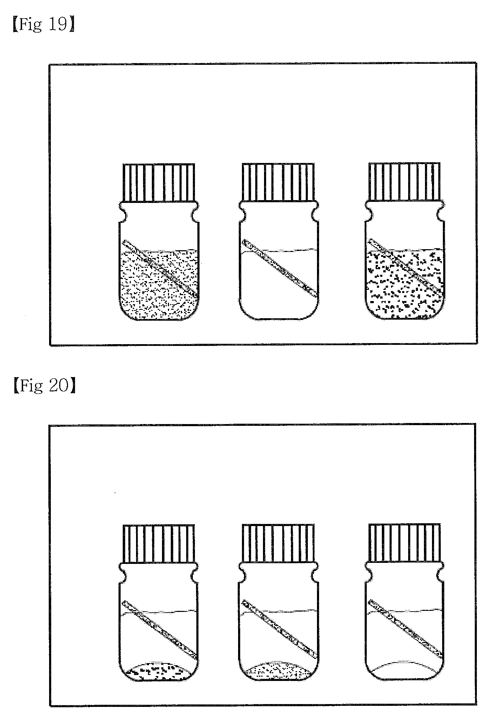

A (left part in FIG. 19): tap water
B (central part in FIG. 19): 5 ceramic balls (9gm)
C (right part in FIG. 19): ceramic powder 1gm 80 ml water put into 100 ml capacity bottle bottle + water weight

2. Results

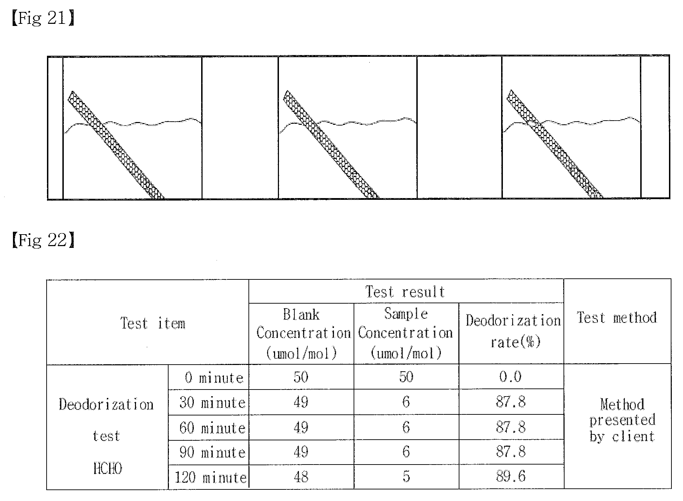

As shown in FIG. 20 and FIG. 21, mineral somatid exhibited an anti-oxidation effect as follows:

A: Red rust appeared on the nail surface as usual, and the size of the rust relatively increased. Rust particles increased in size and dropped on the bottom. Rust particles around the nail looked as if they were connected by spider's threads, and moved as if they were connected by threads when they were lightly touched.
B: The rusting speed was slower, and the size of rust was smaller. Not much rust appeared to be connected. Red rust accumulated on the bottom. The nail surface was black.
C: The rusting speed was the slowest, a small amount of rust occurred, and the size of rust particles was the smallest. No rust floated on the surface.

**[Experiment 8]**

Clinical trial was performed on patients with severe diseases in order to examine enhanced immune activity due to bedding and clothes made of fiber to which the mineral somatid according to the present invention is added.

1. Selection Criteria

(1) Subjects (including inpatients) who voluntarily agreed to participate in the clinical trial and signed on an agreement of the handling of subject information
(2) At least 20-years old male/female subjects
(3) Subjects with life expectancy of at least 6 months
(4) Subjects staying in hospital with severe diseases

2. Method of Statistical Analysis

(1) First effectiveness evaluation

After treated with a clinical trial material, Paired T-Test or Wilcoxon Signed Rank Test was performed at a positive level of significance of 5% in order to inspect whether changes in CD4, CD25 and Fox P3 (sex factor) on weeks 16 (visit 4) with respect to the baseline (visit 1) were statistically significant.

(2) Safety

The number and percentage of subjects who experienced any of abnormalities reported to them was recorded. The number and percentage of subjects were set according to the seriousness of abnormalities, the relevance to the clinical trial material, an orthodontic treatment and its result.
The results of laboratory examination and vital signs were set according to the average, standard deviation, minimum and maximum. The difference between before and after the treatment using the clinical trial material was inspected by Paired T-Test or Wilcoxon Signed Rank Test.

3. Conclusion

In this clinical trial performed on patients with severe diseases using bedding and closes containing the mineral somatid, an improvement in immune cells (CD4, CD25, Fox P3) was confirmed.

During 4-month period, all of the typical immune cells CD4, CD25 and Fox P3 increased from before the experiment, which is statistically significant.

**[Experiment 9]**

VOC deodorization and ammonia deodorization experiments using mineral somatid according to the present invention were performed.

1. VOC Deodorization Experiment

(1) Experiment Method

1. A 20g sample presented by a client was put into a reactor having a 5L size, and the reactor was sealed.
2. An experimental gas was blown by an initial concentration of $50\mu$mol/mol. The concentration of the experimental gas was measured at the initial stage (0 minute), 30 minutes, 60 minutes, 90 minutes and 120 minutes. The measured concentrations were referred to as the concentrations of the sample.
3. The concentration of the experimental gas was measured according to KS I 2218: 2009.
4. During the experiment, temperature was maintained at 23°C±5°C, and relative humidity was maintained at 50%±10%.
5. The above-mentioned processes B to D were performed without the sample. The resultant concentration was referred to as a blank concentration.
6. The removal rate of the experimental gas per time is calculated by the following formula:

$$\text{Experimental gas removal rate } (\%) = [\{\text{blank concentration}\} - (\text{sample concentration})\}/(\text{blank concentration})]$$

2. Results

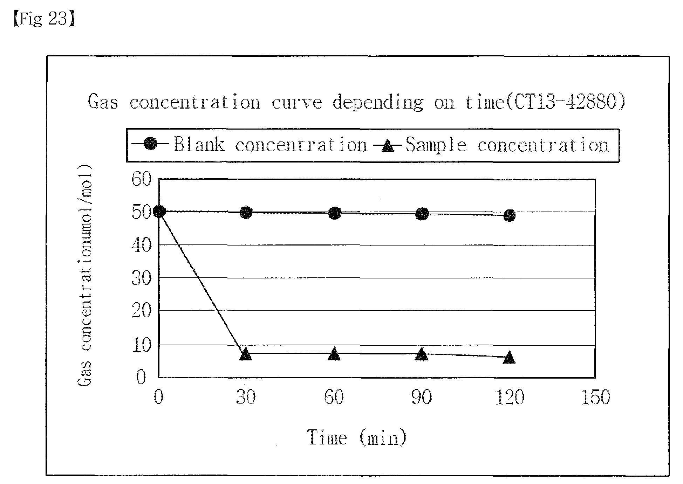

As shown in FIG. 22 and FIG. 23, the VOC deodorization experiment using the mineral somatid according to the present invention showed a deodorization rate of 87.8% after 30 minutes and a deodorization rate of 89.6% after 120 minutes.

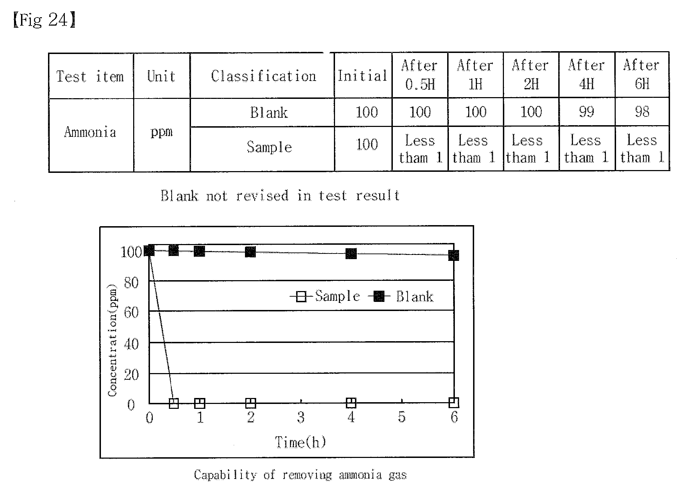

As for the ammonia deodorization experiment, as shown in FIG. 24, it was observed that a significant change in the concentration occurred after 0.5 hour, i.e. the initial concentration of 100 ppm changed to be less than 1 ppm.

Accordingly, it can be appreciated that the mineral somatid according to the present invention has a significant effect on the deodorization of ammonia.

**[Experiment 10]**

Experiment 10 was carried out on an electromagnetic radiation absorber which was made into a flat shape by mixing carbon, pine resin and yellow earth. The ability of the electromagnetic radiation absorber to absorb electromagnetic radiation was examined using a simplified apparatus according to Time Domain Reflectivity Test. The ability of the electromagnetic radiation absorber to block electromagnetic radiation was also examined using a shielding apparatus according to IEEE Std 299 & MIL-STD-188-125 about the electromagnetic shielding rate.

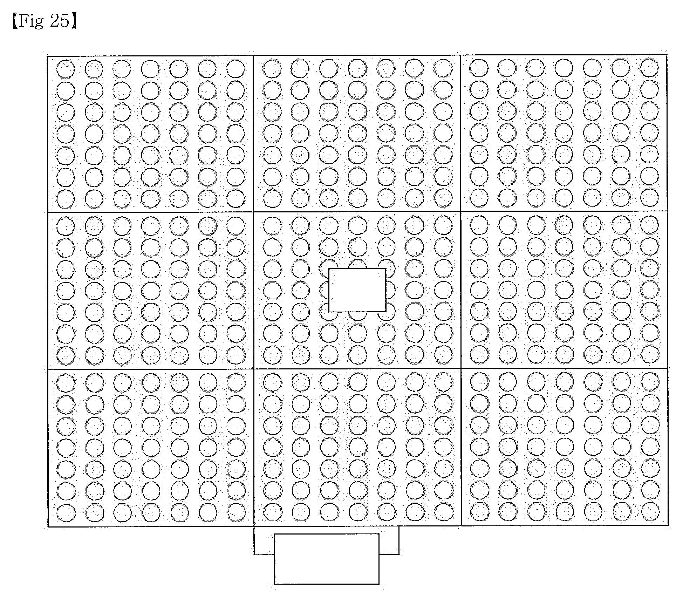

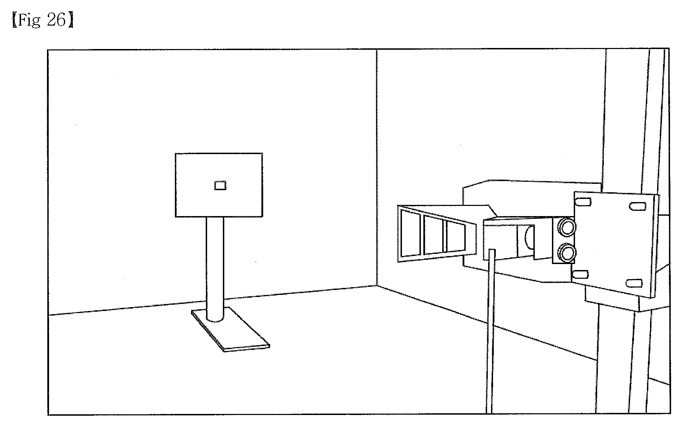

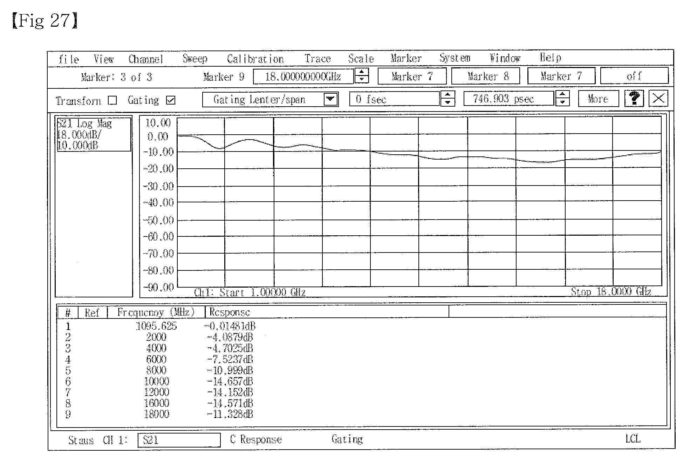

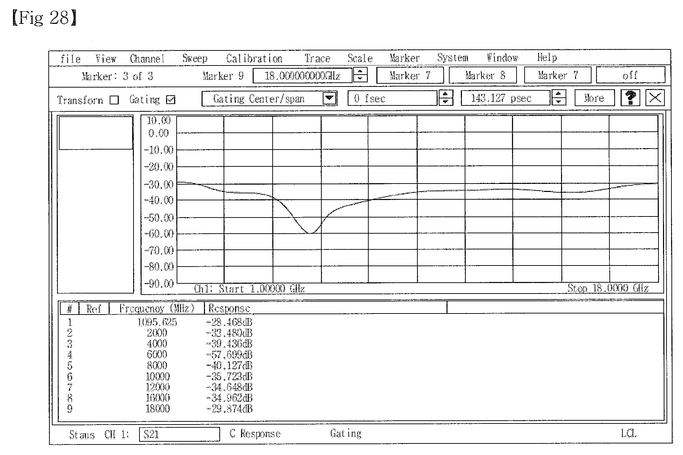

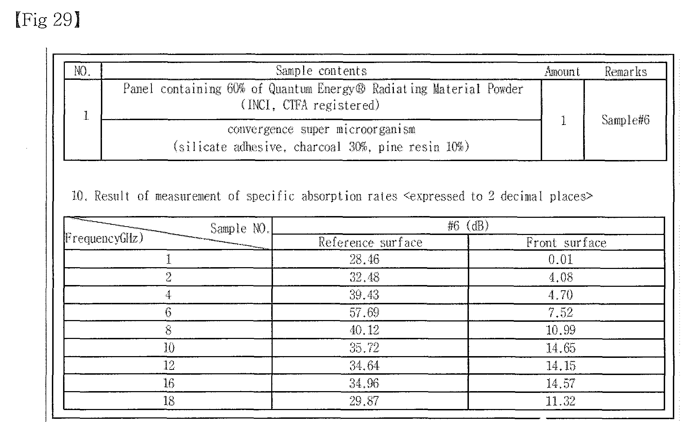

In Experiment 10, the ability to absorb electromagnetic radiation was measured using a panel, as shown in FIG. 25 and FIG. 26. The panel contains 60% of the mineral somatid according to the present invention, 30% of charcoal and 10% of pine resin. According to the experimental result, the panel had specific absorption rates shown in the graphs and table of FIG. 27 to FIG. 29.

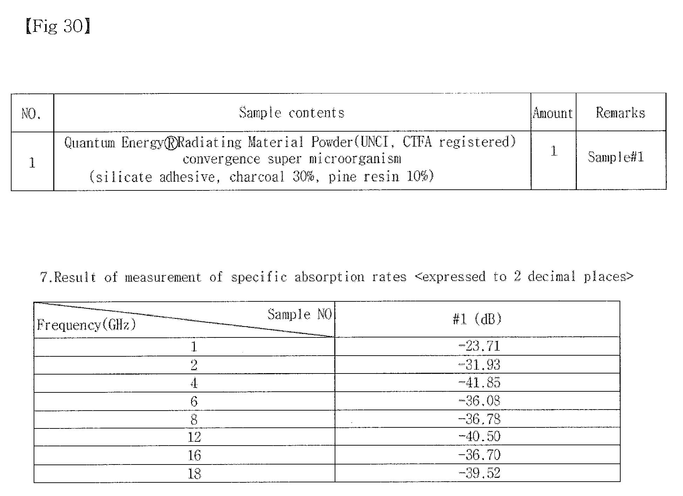

In addition, as shown in FIG. 30, a panel containing 60% of the mineral somatid, 35% of carbon and 5% or pine resin was used. According to the experimental result, the panel had specific absorption rates as shown in the graphs of FIG. 25 and FIG. 26.

**[Experiment 11]**

Experiment 11 was performed in order to test the mineral somatid according to the present invention following "L610. Snow removal agent" method. The heat-generating performance of the mineral somatid was tested by examining whether or not it can melt ice at -12°C and -5°C.

- Process

A) A dish was washed with an organic solvent such as ethanol in order to remove oil or grease, and the washed dish was completely dried.
B) Distilled water or deionized water 130 ml was put on the dish, and the dish was turned or shaken so that water is dispersed over the entire surface of the dish.
C) The dish was put on a horizontal plane of a temperature-adjustable thermostat (see 5.1.3).
D) When water was completely frozen, the surface of the ice was melted to be flat using an Al disk having a thickness of 12.7 mm (a diameter of about 21.6 mm).
E) The dish was put into a low-temperature thermostat, and water was refrozen so as to be in equilibrium with a prescribed working temperature.

- Separation, Measurement and Addition of Experimental Sample

A) The weight of the experimental sample (mineral somatid) 10±0.1g was measured using an analytical balance (see 6.1.2).
B) The weight-measured sample was uniformly laid on the glass that was prepared according to 5.2.1.

- Examination of Melted Ice

A) When the experimental sample was laid on a test piece, a timer was started.
B) Pictures were taken after 24 hours, 48 hours and 72 hours, and the melted state of the ice was examined.

- Repetition of Experiment

Experiment was performed three times for each experimental sample at prescribed temperatures.

- Results

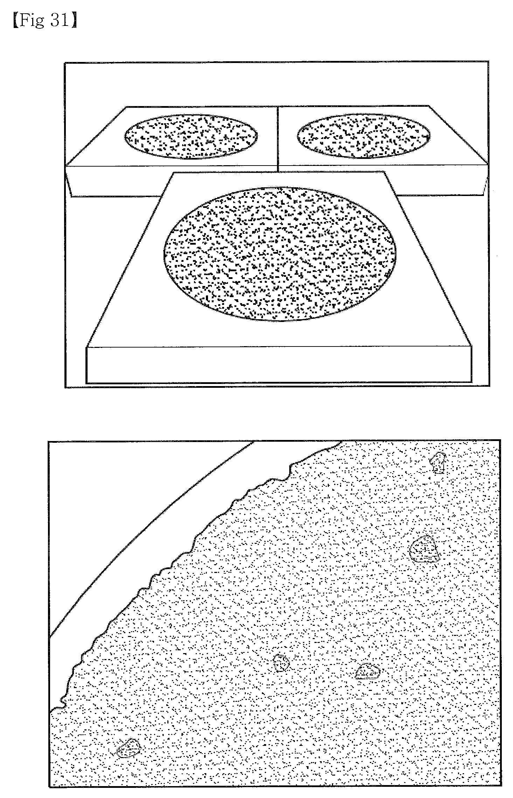

As shown in FIG. 31, the experimental sample failed to completely melt the ice under a condition of -12°C. However, when the sample was inspected, it can be appreciated that sample particles formed round agglomerates.

**[Experiment 12]**

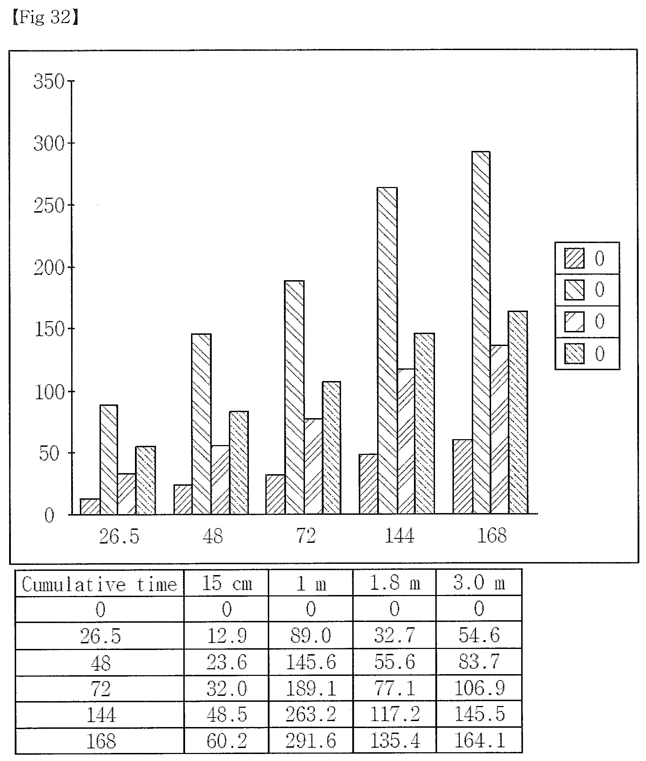

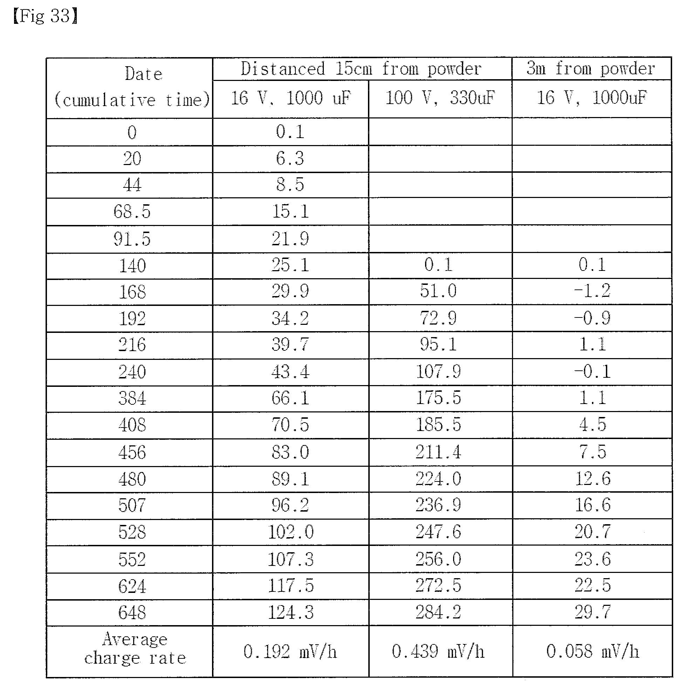

Experiment 12 was performed to examine the current discharge effect of the mineral somatid. As shown in FIG. 32 to FIG. 34, it can be appreciated that an electrical capacitor discharged at 0.1 mV or below was being charged by the mineral somatid.
Specifically, capacitors were distanced 15 cm (purple), 1 m (reddish purple), 1.8 m (light yellow) and 3.0 m (light blue) from the mineral somatid according to the present invention. The capacitors were examined as to whether or not they were charged for certain times. Consequently, it can be appreciated that the capacitors were being charged with a current ranging from 12.9 to 164.1 mV for 26.5 hours to 168 hours.

**[Experiment 13]**

In Experiment 13, the possibility of microbial fusion of mineral powder from among mineral somatid, which has undergone high-temperature pretreatment processing, was inspected, the potential existence of microbial species were investigated and identified, and antibacterial ability against various kinds of pathogenic bacteria was qualitatively and quantitatively tested.

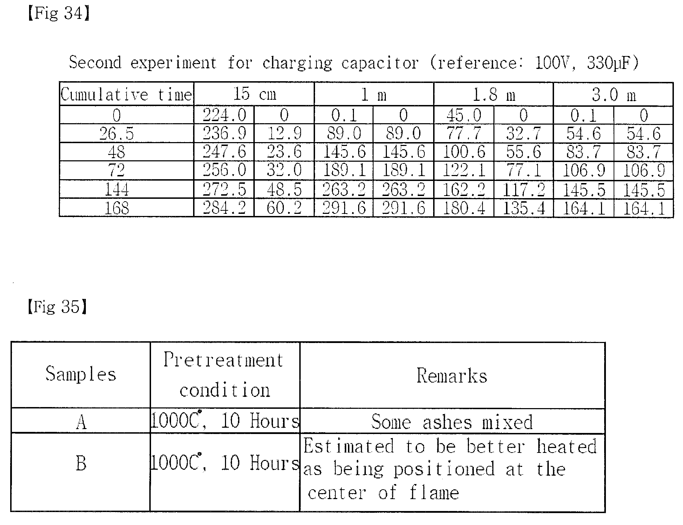

In order to investigate and analyze microbial species contained in the mineral powder, as shown in FIG. 35, two types of samples (Sample A and Sample B) were tested after pretreatment processing.

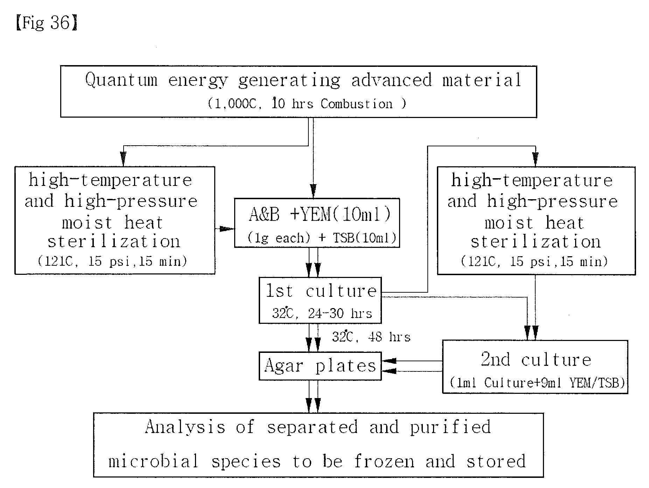

After the pretreatment, microorganisms included in Sample A and Sample B were cultured. FIG. 36 is a schematic diagram showing the procedure with which microbial species were investigated, separated and identified.

Generally known minimum and complex nutrient media were used as microbial nutrient media for studying the possibility of microbial fusion of the mineral powder in which mineral somatid QELBs and a number of microbial species were included.

The minimum nutrient media were yeast extraction mineral media (YEM), which were used after sterilization. The composition of the YEM consisted of $Na_2HPO_4 \cdot 12H_2O$ 3.5 g, $K_2HPO_4$ 1.0 g, $MgSO_4 \cdot 7H_2O$ 0.03g, $NH_4Cl$ 0.5 g, Yeast extract 4.0 g, distilled water (D.W.) up to 1L.

Tryptic Soy Broth (TSB, available from BD & Company) was used as the complex nutrient media, and solid media were prepared by adding 1.5% (w/v) Bacto-agar to the compositions of YEM and TSB.

Microbial culture was maintained at 32°C for 24 to 72 hours by aerobic culture and anaerobic culture. For liquid aerobic culture, a shaking incubator was used as an attempt to culture microorganisms at 250 rpm. For anaerobic culture, an automatic anaerobic and microaerobic bacterial culture system (Anoxomat Mark II System) was used.

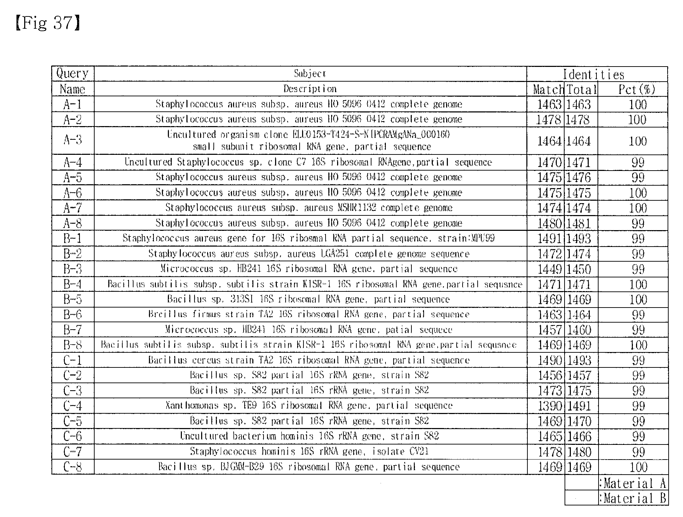

As shown in FIG. 37, the cell shapes of all of separated microbial species were observed using a microscope in order to identify the microbial species. Genomic DNAs were separated and purified using a CTAB method, and were studied using a BLAST method (http://blast.ncbi.nlm.nih.gov/Blast.cgi).

As the result of the experiment, a total 53 kinds of aerobic microorganisms harmless to living bodies was separated and purified from Sample A and Sample B. Among the total aerobic microorganisms, 24 kinds were subjected to molecular genetic identification. In the 24 kinds, 13 kinds belonged to Sample A and 11 kinds belonged to Sample B, the samples being mineral powder samples. It was proved that 3 microbial species were separated from Sample A and Sample B, which were subjected to high-temperature and high-pressure moist heat sterilization, whereas 21 kinds were separated from Sample A and Sample B before the sterilization.

It was also proved that small microorganisms having a size of 1 $\mu$m were present in the microorganisms separated from Sample A and Sample B before the sterilization. As a notable feature, an object having the shape of a thin film was formed on the bottom of a Petri dish used in the culture. This result is similar to that of the experiment at step S340.

In particular, as an attempt to find an error in the experiment, cultured microorganisms were separated, sterilized in an autoclave at 121°C for 15 minutes, and then subjected to re-culture, but the microorganisms did not grow. In contrast, when uncultured environmental microorganisms were put into a medium together with natural powder of mineral somatid QELBs, sterilized at 121°C for 15 minutes, and then subjected to re-culture, the culture of the microorganisms was observed.

These results indicate that the mineral somatid QELBs act to protect and save common microorganisms. In contrast, when separated microorganisms were subjected to culture after being sterilized under a pressure without the addition of the mineral somatid QELBs, there was no culture of the microorganisms since all of the microorganisms died.

This indicates that the natural powder of the mineral somatid QELBs provide shelters where common environmental microorganisms can survive in the high-temperature environment or act to energize the common environmental microorganisms such that the microorganisms can survive in the high-temperature environment.

The foregoing exemplary embodiments were presented for the purposes of illustrating the principles of the present invention. A person having ordinary skill in the art can make various changes and alterations thereof without departing from the spirit of the present invention. Accordingly, the foregoing embodiments should be regarded as illustrative rather than limiting the principle of the present invention, and the scope of the present invention is not defined by the foregoing embodiments. It should be understood that the scope of the present invention shall be interpreted by the appended Claims, and that all technical ideas equivalent to the Claims shall be construed to fall within the scope of the present invention.

## INDUSTRIAL APPLICABILITY

As set forth above, the present invention can separate mineral somatid from the mineral, culture the separated mineral somatid, and combine the cultured mineral somatid with ceramic powder. The composite material according to the present invention is harmless, has no biological side effects, and has multiple functions including cancer suppression, immune enhancement, skin reconstruction, self-heating, cold activeness, VOC deodorization and harmful electromagnetic radiation shielding and absorption.

In addition, the advanced multifunctional composite material can be mass-produced more easily and at a low cost by mixing QELBs (mineral somatid) that show active signs of life and have multiple functions, such as healing, with ceramic powder made of various kinds of microbial ceramic materials which can persist when heated at a high temperature of 1,000°C for 10 hours. The multifunctional composite material in which the mineral somatid having superior dispersibility is contained can be easily combined with fiber and a polymeric material, such as chemicals. It is also possible to develop new composite materials by combining the multifunctional composite material with industrial materials such as iron, nonferrous materials and ceramics.

Furthermore, the mineral somatid according to the present invention acts to neither attack nor kill cells or microorganisms that are beneficial or not harmful to living bodies while protecting them to survive at high temperature or in the hostile environment. The mineral somatid also suppresses pathogenic bacteria that are harmful to living bodies or super bacteria resistant to antibiotics (antibiosis). Furthermore, the mineral somatid has the functions of healing the outer and inner skins of a patient who is suffering from wound, bedsore or the like, suppressing cancer cells, improving immunity, enhancing homeostasis that allows living bodies to maintain health, and increasing resistance to diseases.

In addition, the mineral somatid according to the present invention can serve as catalyst that enhances the intrinsic properties of a material by being combined with a variety of materials, such as polymeric materials, metal materials, inorganic materials or organic materials. The mineral somatid is a material that suppresses oxidation but enables reduction.

**Claims**

1. A method of extracting mineral somatid, comprising:

   (a) mining a natural mineral from the earth, wherein the natural mineral includes inorganic matters of $SiO_2$, $Al_2O_3$, $Fe_2O_3$ and $MgO_3$, does not have heavy meals or a radioactive substance harmful to living bodies, and is not contaminated;
   (b) crushing the mined natural mineral into powder having a particle size ranging from 320 meshes to 2 nanometers;
   (c) refining the mineral powder by removing heavy metals and radioactive substances that are harmful to living bodies from the mineral powder;
   (d) burning the refined mineral powder in order to change a mass, a specific gravity, a number of electrons and an ion number of the refined mineral powder;
   (e) mixing the burned mineral powder with $H_2O$ and a natural plant extract made of agar at certain ratios and fermenting a mineral powder mixture for a predetermined period so that mineral somatid and microorganisms are activated;
   (f) sterilizing and drying the fermented mineral powder mixture;
   (g) putting the dried mineral powder mixture into a container, adding a solution that includes a predetermined amount of water, distilled water, magnetized water, glucose or sugar to the mineral powder mixture, adding sugar or a nutrient medium for culturing of microorganisms to the mineral powder mixture to which the solution is added, and culturing the mineral powder mixture for at least one hour; and
   (h) extracting microorganisms from the mineral powder mixture cultured at the step (g) and extracting mineral somatid from the extracted microorganisms.

2. The method of claim 1, wherein the step (d) of burning the refined mineral powder comprises:

   a first burning process of calcining the mineral powder by heating the mineral powder at a temperature ranging from 50 to 300°C for 2 to 3 hours; and
   after the first burning process is completed, a second burning process of burning the mineral powder that has been burned in the first burning process by heating the mineral powder at a temperature ranging from 300 to 850°C for 30 minutes to 10 hours.

3. The method of claim 1, wherein the step (e) of mixing the burned mineral powder comprises forming the mineral powder mixture by mixing, by weight, 70% the mineral powder that has been crushed at a size ranging from 320 meshes to 2 nanometers, 25% $H_2O$ and 5% a nontoxic plant extract of roots, stems or leaves.

4. The method of claim 1, wherein the step (e) of fermenting the mineral powder mixture comprises putting the mineral powder mixture into a container, and ripening and fermenting the mineral powder mixture in a ripening chamber at a temperature ranging from -10 to 200°C for 10 to 90 days so that the mineral somatid and microorganisms are activated.

5. The method of claim 1, wherein the mineral powder of the mineral powder mixture is crushed until a size of the mineral powder ranges from 320 meshes to 2 nanometers so that only the powder from which heavy metals and harmful components are removed is extracted for use.

6. The method of claim 1, wherein sterilizing and drying the fermented mineral powder mixture comprises rotating and sterilizing the mineral powder mixture inside a calciner at a high temperature of 180°C or below for 30 minutes to 2 hours, and drying the sterilized mineral powder mixture at a temperature ranging from 150 to 200°C.

7. The method of claim 1, wherein conditions for culturing the mineral powder mixture include a culture medium selected from the group consisting of yeast extraction mineral (YEM) medium, tryptic soy broth (TSB) medium, M9 medium and Luria broth (LB) medium and a culturing temperature ranges from 30 to 37°C, a composition of yeast extraction mineral (YEM) in the medium consists of $Na_2HPO_4 \cdot 12H_2O$ 3.5g, $K_2HPO_4$ 1.0g, $MgSO_4 \cdot 7H_2O$ 0.03g, $NH_4Cl$ 0.5g, yeast extract 4.0g, agar 15.0g, and distilled water 1.0L.

8. The method of claim 1, wherein extracting the mineral somatid comprises extracting only mineral somatid that continues life activity and radiates energy after being sterilized at a high temperature of 1,000°C or higher for at least 10 hours in an autoclave.

【Fig 1】

```
              START

          Mining Step            ── S110

       Mineral Crushing Step     ── S120

    Mineral Powder Crushing Step ── S130

    Mineral Powder Burning Step  ── S140

     Mixing And Fermenting Step  ── S150

     Sterilizing And Drying Step ── S160

          Culturing Step         ── S170

    Microorganism Separating And
         Extracting Step         ── S180

   Mineral Somatid Separating And
         Extracting Step         ── S190

               END
```

【Fig 2】

【Fig 3】

【Fig 4】

【Fig 5】

(a)

(b)        (c)        (d)        (e)

【Fig 6】

【Fig 7】

【Fig 8】

【Fig 9】

【Fig 10】

Mineral Somatid                    Ge                         Trast

【Fig 11】

EP 3 009 415 A1

【Fig 12】

| | No. | score | | Number of individuals with symptoms alleviated/total number of individuals |
|---|---|---|---|---|
| | | day 0 | day 30 | |
| Group not treated After induction | 1 2 | 11 8 | 12 9 | 0/2 |
| Mineral somatid Treatment group | 1 2 3 4 5 | 4 8 8 12 4 | 12 6 6 12 7 | 2/5 |
| Ge Treatment group | 1 2 3 4 5 | 11 12 10 9 4 | 8 12 11 7 5 | 2/5 |
| Trast treatment group | 1 2 3 4 5 | 12 5 6 11 1 | 9 3 2 8 1 | 4/5 |

【Fig 13】

Not induced    Mineral SomatidMineral Somatid    Trast

Induced and TreatedInduced and Not treated    Ge

32

【Fig 14】

| Type II collagen | Nitrite concentrations (NO) | | | | |
|---|---|---|---|---|---|
| | Not induced | Induced and Not treated | Mineral SomatidMineral Somatid | Ge | Trast |
| 0 ul/ml | 0.068±0.004 | 0.071±0.005 | 0.062±0.006 | 0.059±0.006 | 0.064±0.006 |
| 200 ul/ml | 0.067±0.002 | 0.069±0.012 | 0.059±0.004 | 0.062±0.005 | 0.068±0.009 |

【Fig 15】

| | No. | Weight | | Number of individuals With increased Weight/total number Of individuals |
|---|---|---|---|---|
| | | day 0 | day 21 | |
| Group fed With general Feedstuff | 1 | 30.4 | 28.4 | 3/7 |
| | 2 | 23.4 | 23.1 | |
| | 3 | 27 | 28.7 | |
| | 4 | 33.5 | 32.3 | |
| | 5 | 25.7 | 26.8 | |
| | 6 | 24.3 | 23.1 | |
| | 7 | 21.8 | 23.5 | |
| Group Fed with Mineral Somatid-added Feedstuff | 1 | 23 | 23.9 | 5/7 |
| | 2 | 24.2 | 26.9 | |
| | 3 | 28.1 | 27.7 | |
| | 4 | 23.3 | 24.7 | |
| | 5 | 28 | 24.2 | |
| | 6 | 27.8 | 29.8 | |
| | 7 | 22.6 | 25.5 | |

【Fig 16】

Legend:
- Normal group
- Mineral somatidMineral somatid
- Common cloth
- Control group

【Fig 17】

Legend:
- Normal group
- Mineral somatidMineral somatid
- Common cloth
- Control group

[Fig 18]

【Fig 19】

【Fig 20】

【Fig 21】

【Fig 22】

| Test item | | Test result | | | Test method |
|---|---|---|---|---|---|
| | | Blank Concentration (umol/mol) | Sample Concentration (umol/mol) | Deodorization rate(%) | |
| Deodorization test HCHO | 0 minute | 50 | 50 | 0.0 | Method presented by client |
| | 30 minute | 49 | 6 | 87.8 | |
| | 60 minute | 49 | 6 | 87.8 | |
| | 90 minute | 49 | 6 | 87.8 | |
| | 120 minute | 48 | 5 | 89.6 | |

【Fig 23】

Gas concentration curve depending on time(CT13-42880)

Blank concentration —▲—Sample concentration

Gas concentration(umol/mol) vs Time (min)

【Fig 24】

| Test item | Unit | Classification | Initial | After 0.5H | After 1H | After 2H | After 4H | After 6H |
|---|---|---|---|---|---|---|---|---|
| Ammonia | ppm | Blank | 100 | 100 | 100 | 100 | 99 | 98 |
| | | Sample | 100 | Less tham 1 | Less tham 1 | Less tham 1 | Less tham 1 | Less tham 1 |

Blank not revised in test result

Capability of removing ammonia gas

【Fig 25】

【Fig 26】

【Fig 27】

【Fig 28】

【Fig 29】

| NO. | Sample contents | | Amount | Remarks |
|---|---|---|---|---|
| 1 | Panel containing 60% of Quantum Energy® Radiating Material Powder (INCI, CTFA registered) | | 1 | Sample#6 |
| | convergence super microorganism (silicate adhesive, charcoal 30%, pine resin 10%) | | | |

10. Result of measurement of specific absorption rates <expressed to 2 decimal places>

| Frequency(GHz) Sample NO. | #6 (dB) | |
|---|---|---|
| | Reference surface | Front surface |
| 1 | 28.46 | 0.01 |
| 2 | 32.48 | 4.08 |
| 4 | 39.43 | 4.70 |
| 6 | 57.69 | 7.52 |
| 8 | 40.12 | 10.99 |
| 10 | 35.72 | 14.65 |
| 12 | 34.64 | 14.15 |
| 16 | 34.96 | 14.57 |
| 18 | 29.87 | 11.32 |

【Fig 30】

| NO. | Sample contents | Amount | Remarks |
|---|---|---|---|
| 1 | Quantum Energy®Radiating Material Powder(UNCI, CTFA registered) convergence super microorganism (silicate adhesive, charcoal 30%, pine resin 10%) | 1 | Sample#1 |

7.Result of measurement of specific absorption rates <expressed to 2 decimal places>

| Frequency(GHz) Sample NO | #1 (dB) |
|---|---|
| 1 | -23.71 |
| 2 | -31.93 |
| 4 | -41.85 |
| 6 | -36.08 |
| 8 | -36.78 |
| 12 | -40.50 |
| 16 | -36.70 |
| 18 | -39.52 |

【Fig 31】

【Fig 32】

| Cumulative time | 15 cm | 1 m | 1.8 m | 3.0 m |
|-----------------|-------|------|-------|-------|
| 0               | 0     | 0    | 0     | 0     |
| 26.5            | 12.9  | 89.0 | 32.7  | 54.6  |
| 48              | 23.6  | 145.6| 55.6  | 83.7  |
| 72              | 32.0  | 189.1| 77.1  | 106.9 |
| 144             | 48.5  | 263.2| 117.2 | 145.5 |
| 168             | 60.2  | 291.6| 135.4 | 164.1 |

【Fig 33】

| Date (cumulative time) | Distanced 15cm from powder | | 3m from powder |
|---|---|---|---|
| | 16 V, 1000 uF | 100 V, 330uF | 16 V, 1000uF |
| 0 | 0.1 | | |
| 20 | 6.3 | | |
| 44 | 8.5 | | |
| 68.5 | 15.1 | | |
| 91.5 | 21.9 | | |
| 140 | 25.1 | 0.1 | 0.1 |
| 168 | 29.9 | 51.0 | −1.2 |
| 192 | 34.2 | 72.9 | −0.9 |
| 216 | 39.7 | 95.1 | 1.1 |
| 240 | 43.4 | 107.9 | −0.1 |
| 384 | 66.1 | 175.5 | 1.1 |
| 408 | 70.5 | 185.5 | 4.5 |
| 456 | 83.0 | 211.4 | 7.5 |
| 480 | 89.1 | 224.0 | 12.6 |
| 507 | 96.2 | 236.9 | 16.6 |
| 528 | 102.0 | 247.6 | 20.7 |
| 552 | 107.3 | 256.0 | 23.6 |
| 624 | 117.5 | 272.5 | 22.5 |
| 648 | 124.3 | 284.2 | 29.7 |
| Average charge rate | 0.192 mV/h | 0.439 mV/h | 0.058 mV/h |

【Fig 34】

Second experiment for charging capacitor (reference: 100V, 330μF)

| Cumulative time | 15 cm | | 1 m | | 1.8 m | | 3.0 m | |
|---|---|---|---|---|---|---|---|---|
| 0 | 224.0 | 0 | 0.1 | 0 | 45.0 | 0 | 0.1 | 0 |
| 26.5 | 236.9 | 12.9 | 89.0 | 89.0 | 77.7 | 32.7 | 54.6 | 54.6 |
| 48 | 247.6 | 23.6 | 145.6 | 145.6 | 100.6 | 55.6 | 83.7 | 83.7 |
| 72 | 256.0 | 32.0 | 189.1 | 189.1 | 122.1 | 77.1 | 106.9 | 106.9 |
| 144 | 272.5 | 48.5 | 263.2 | 263.2 | 162.2 | 117.2 | 145.5 | 145.5 |
| 168 | 284.2 | 60.2 | 291.6 | 291.6 | 180.4 | 135.4 | 164.1 | 164.1 |

【Fig 35】

| Samples | Pretreatment condition | Remarks |
|---|---|---|
| A | 1000C°, 10 Hours | Some ashes mixed |
| B | 1000C°, 10 Hours | Estimated to be better heated as being positioned at the center of flame |

[Fig 36]

【Fig 37】

| Query | Subject | | Identities | | |
|---|---|---|---|---|---|
| Name | Description | | Match | Total | Pct(%) |
| A-1 | Staphylococcus aureus subsp. aureus HO 5096 0412 complete genome | | 1463 | 1463 | 100 |
| A-2 | Staphylococcus aureus subsp. aureus HO 5096 0412 complete genome | | 1478 | 1478 | 100 |
| A-3 | Uncultured organism clone ELU0153-T424-S-NIPCRAMgANa_000160 small subunit ribosomal RNA gene, partial sequence | | 1464 | 1464 | 100 |
| A-4 | Uncultured Staphylococcus sp. clone C7 16S ribosomal RNAgene, partial sequence | | 1470 | 1471 | 99 |
| A-5 | Staphylococcus aureus subsp. aureus HO 5096 0412 complete genome | | 1475 | 1476 | 99 |
| A-6 | Staphylococcus aureus subsp. aureus HO 5096 0412 complete genome | | 1475 | 1475 | 100 |
| A-7 | Staphylococcus aureus subsp. aureus MSHR1132 complete genome | | 1474 | 1474 | 100 |
| A-8 | Staphylococcus aureus subsp. aureus HO 5096 0412 complete genome | | 1480 | 1481 | 99 |
| B-1 | Staphylococcus aureus gene for 16S ribosmal RNA partial sequence, strain:MPU99 | | 1491 | 1493 | 99 |
| B-2 | Staphylococcus aureus subsp. aureus LGA251 complete genome sequence | | 1472 | 1474 | 99 |
| B-3 | Micrococcus sp. HB241 16S ribosomal RNA gene, partial sequence | | 1449 | 1450 | 99 |
| B-4 | Bacillus subtilis subsp. subtilis strain KISR-1 16S ribosomal RNA gene, partial sequsnce | | 1471 | 1471 | 100 |
| B-5 | Bacillus sp. 313SI 16S ribosomal RNA gene, partial sequence | | 1469 | 1469 | 100 |
| B-6 | Brcillus firmus strain TA2 16S ribosomal RNA gene, partial sequence | | 1463 | 1464 | 99 |
| B-7 | Micrococcus sp. HB241 16S ribosomal RNA gene, patial sequece | | 1457 | 1460 | 99 |
| B-8 | Bacillus subtilis subsp. subtilis strain KISR-1 16S ribosomal RNA gene, partial sequsnce | | 1469 | 1469 | 100 |
| C-1 | Bacillus cereus strain TA2 16S ribosomal RNA gene, partial sequence | | 1490 | 1493 | 99 |
| C-2 | Bacillus sp. S82 partial 16S rRNA gene, strain S82 | | 1456 | 1457 | 99 |
| C-3 | Bacillus sp. S82 partial 16S rRNA gene, strain S82 | | 1473 | 1475 | 99 |
| C-4 | Xanthomonas sp. TE9 16S ribosomal RNA gene, partial sequence | | 1390 | 1491 | 99 |
| C-5 | Bacillus sp. S82 partial 16S rRNA gene, strain S82 | | 1469 | 1470 | 99 |
| C-6 | Uncultured bacterium hominis 16S rRNA gene, strain S82 | | 1465 | 1466 | 99 |
| C-7 | Staphylococcus hominis 16S rRNA gene, isolate CV21 | | 1478 | 1480 | 99 |
| C-8 | Bacillus sp. BJGMM-B29 16S ribosomal RNA gene, partial sequence | | 1469 | 1469 | 100 |

:Material A
:Material B

## INTERNATIONAL SEARCH REPORT

| International application No. |
| --- |
| **PCT/KR2014/004877** |

**A. CLASSIFICATION OF SUBJECT MATTER**

*C04B 33/04(2006.01)i, C04B 33/13(2006.01)i, C04B 33/32(2006.01)i*

According to International Patent Classification (IPC) or to both national classification and IPC

**B. FIELDS SEARCHED**

Minimum documentation searched (classification system followed by classification symbols)

C04B 33/04; A61K 36/18; A61H 33/00; A01G 1/00; C12N 1/00; A61H 33/06; B01D 11/02; A23K 1/175; A61K 35/02; A61K 35/14; C04B 33/13; C04B 33/32

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: somatid

**C. DOCUMENTS CONSIDERED TO BE RELEVANT**

| Category* | Citation of document, with indication, where appropriate, of the relevant passages | Relevant to claim No. |
| --- | --- | --- |
| A | JP 2006-166738 A (APATITE CORPORATION:KK et al.) 29 June 2006<br>See the entire document. | 1-8 |
| A | JP 2007-297312 A (HAI, Daichin et al.) 15 November 2007<br>See the entire document. | 1-8 |
| A | JP 2006-321774 A (KOJO:KK et al.) 30 November 2006<br>See the entire document. | 1-8 |
| A | JP 2012-085990 A (OGIWARA, Masaru et al.) 10 May 2012<br>See the entire document. | 1-8 |
| A | JP 2007-306829 A (NOGAMI, Michiko) 29 November 2007<br>See the entire document. | 1-8 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 20 AUGUST 2014 (20.08.2014) | **28 AUGUST 2014 (28.08.2014)** |

| Name and mailing address of the ISA/KR | Authorized officer |
| --- | --- |
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (July 2009)

International application No.

**PCT/KR2014/004877**

| Patent document cited in search report | Publication date | Patent family member | Publication date |
|---|---|---|---|
| JP 2006-166738 A | 29/06/2006 | NONE | |
| JP 2007-297312 A | 15/11/2007 | NONE | |
| JP 2006-321774 A | 30/11/2006 | NONE | |
| JP 2012-085990 A | 10/05/2012 | NONE | |
| JP 2007-306829 A | 29/11/2007 | NONE | |

Form PCT/ISA/210 (patent family annex) (July 2009)

**EP 3 009 415 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006166738 A